# FASCICULE DE BREVET EUROPEEN

(11) **EP 4 007 639 B1**
(45) Date de publication et mention de la délivrance du brevet: **28.01.2026**
(21) Numéro de dépôt: 20775190.0
(22) Date de dépôt: 02.09.2020
(51) Int. Cl.: A61P 35/00, A61K 31/498, C07D 241/46, A61K 41/00

(54) **DÉRIVÉ DE PHÉNAZINE ET SON UTILISATION POUR LE TRAITMENT DU CANCER**
PHENAZINDERIVATE UND IHRE VERWENDUNG ZUR BEHANDLUNG VON KREBS
PHENAZINE DERIVATIVE AND USE THEREOF FOR THE TREATMENT OF CANCER

(30) Priorité: 02.09.2019 FR 1909642
(43) Date de publication de la demande: 08.06.2022
(73) Titulaire: Centre national de la recherche scientifique, 75016 Paris (FR); Universite d'Aix-Marseille, 13007 Marseille (FR)
(72) Inventeur: SIRI, Olivier, 13720 Belcodène (FR); CHEN, Zhongrui, Beijing, Beijing 100096 (CN)
(74) Mandataire: Novagraaf Technologies
(86) Numéro de dépôt international: PCT/EP2020/074447
(87) Numéro de publication internationale: WO 2021/043813

(56) Documents cités:
- EP-A1- 2 368 885
- WO-A1-2007/016762
- WO-A2-2010/129340
- JP-A- H07 309 000
- SUMAN K ROY ET AL: "Aerial Oxidation of Protonated Aromatic Amines. Isolation, X-ray Structure, and Redox and Spectral Characteristics of N- Containing Dyes", JOURNAL OF ORGANIC CHEMISTRY, vol. 77, no. 22, 30 October 2012 (2012-10-30), Japan, pages 10249 - 10259, XP055743275, ISSN: 0022-3263, DOI: 10.1021/jo3019126
- DATABASE CA [online] CHEMICAL ABSTRACTS SERVICE, COLUMBUS, OHIO, US; 1965, LANTZ, ROBERT ET AL: "Oxidative condensation of "Willstaetter emeraldine" with aniline. Analogous reactions leading to aniline black and structures of these dyes", XP002800819, retrieved from STN Database accession no. 1965:410701
- LANTZ, ROBERT ET AL: "Oxidative condensation of "Willstaetter emeraldine" with aniline. Analogous reactions leading to aniline black and structures of these dyes", BULLETIN DE LA SOCIETE CHIMIQUE DE FRANCE , (3), 816-21 CODEN: BSCFAS; ISSN: 0037-8968, 1965
- DATABASE CA [online] CHEMICAL ABSTRACTS SERVICE, COLUMBUS, OHIO, US; 1996, MIURA, SADAMI ET AL: "Manufacture of composite polyester films with good regulation properties of electric charge", XP002800820, retrieved from STN Database accession no. 1996:128301
- DATABASE REGISTRY [online] CHEMICAL ABSTRACTS SERVICE, COLUMBUS, OHIO, US; 29 August 2004 (2004-08-29), XP002800821, Database accession no. 735245-74-6

## Description

L'invention concerne des dérivés de phénazine et leurs utilisations, notamment leurs utilisations thérapeutiques.

Dans le contexte du traitement des pathologies et notamment des tumeurs, de nouvelles molécules et de nouvelles approches sont en permanence développées et testées.

En particulier, ces dernières années, la thérapie photodynamique a connu un essor en particulier pour le traitement des pathologies cutanées. Toutefois, les composés sensibilisants connus à ce jour ne présentent pas les meilleurs effets, et les taux d'échec sont assez grands. En outre, les longueurs d'ondes des lasers utilisés peuvent générer des effets indésirables et induire des lésions profondes des tissus exposés.

Aussi, existe-t-il un besoin de fournir de nouveaux composés qui seraient efficaces pour ce type de thérapie non invasive.

Une approche est d'utiliser des composés fluorescents capables de cibler spécifiquement les cellules d'intérêt et présentant des propriétés thérapeutiques sous activation.

Or de tels composés sont rares.

Certains dérivés de phénazine sont déjà connus. Divers composés ayant une telle structure de phénazine ont déjà été décrits chez Laursen Et al (chem Rev, 2004, 104 :1663), Beifuss Et al (dessus. Curr. Chem, 2005,77), Terech Et al (J Of Coll. Et entre. Sc, 2006 :633), Llusar Et al (Zeit. Fuer Anorg. Und Allge, chem, 2005, 631 : 2215; J Of Mat. Chem, 2003, 13 : 2505), Pozzo et al (Mol. Les cristaux et les Cristaux liquides Sc Et Tech, 2000, 344 : 101; J Of Mat. Chem, 1998, 8 : 2575) et US 2004/065227. Toutefois, à la connaissance des inventeurs, ces composés n'ont jamais été proposés comme agents chimiothérapeutiques En outre, la demande de brevet WO2011117830 décrit des composés dérivés de la phénazine, mais ici encore à la connaissance des inventeurs de tels composés n'ont pas de propriétés connues pour être utilisés en thérapie photodynamique.

Aussi, il existe toujours un besoin de nouveaux composés, et l'invention vise à pallier ce manque.

L'un des buts de l'invention est de fournir des composés utilisables en thérapie photodynamique qui soient efficaces, peu chers, faciles à produire.

Un autre but de l'invention est de proposer diverses utilisations thérapeutiques et diagnostiques de ces nouveaux composés.

L'invention concerne un composé de formule I suivant : 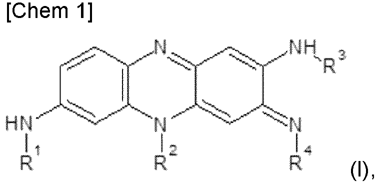 où,
indépendamment l'un de le l'autre, R1 et R2 sont un alkyle en C₄-C₁₀, linéaire ou ramifié, saturé ou non, cyclique ou non, et
indépendamment l'un de l'autre, R3 et R4 sont
   - ou H,
   - ou un alkyle en C1-C18, linéaire ou ramifié, saturé ou non, cyclique ou non, éventuellement substitué par un ou plusieurs groupe choisi parmi un groupe hydroxyl, un groupe amino, un groupe aminoalkyle, un groupe alkoxy en C1-C5, un alkyl en C1-C5, un peptide, un groupe pyridine, un groupe phosphine, un thiol, un C2 alcène, un groupe C2 alcyne et un halogène, ou un radical benzyle éventuellement substitué par un ou plusieurs radicaux choisi parmi un groupe hydroxyl, un groupe amino, un groupe aminoalkyl, un groupe C1-C5 alkoxy, un groupe C1- C5 alkyl, et un atome d'halogène,
   - ou un groupe (hétéro)aryle, éventuellement substitué par un ou plusieurs groupe choisi parmi un groupe hydroxyl, un groupe amino, un groupe amino alkyle, un groupe alkoxy en C1-C5, un alkyl en C1-C5, un peptide, un groupe pyridine, un groupe phosphine, un thiol, un C2 alcène, un groupe C2 alcyne et un halogène, or un radical benzyl éventuellement substitué par un ou plusieurs radicaux choisi parmi un groupe hydroxyl, un groupe amino, un groupe aminoalkyl, un groupe C1-C5 alkoxy, un groupe C1-C5 alkyl, et un halogène,
   - ou encore R3 et R4 sont l'un ou l'autre, ou les deux, un groupe fonctionnel carbonyle formant des fonctions amides incluant des peptides ou non,
ou un sel ou un solvate de celui-ci, ou une forme protonée de celui-ci.

L'invention repose sur la constatation surprenante faite par les inventeurs que les dérivés de phénazine susmentionnés possèdent des propriétés fluorescentes variables selon le degré de protonation pour des études *in vitro* et *in vivo* dans un objectif d'imagerie. Ces molécules sont également utilisables dans le cadre de thérapie photodynamique (PDT), à un et deux photons.

Aussi, la présente invention décrit une famille de composés originaux, peu toxiques et particulièrement prometteuse pour l'imagerie a un et deux photons selon la cible visée. Leur synthèse est réalisée en peu d'étapes et à bas cout. L'intensité des marquages est remarquable, ce qui permet objectivement d'envisager une identification plus précise des cibles cytoplasmiques. Les intenses foyers de fluorescence obtenus dans des régions périnucléaires pourraient notamment correspondre à la localisation de la sonde au niveau du réticulum endoplasmique. Ces composés permettent donc d'envisager une avancée cruciale dans le domaine de l'imagerie sélective, et de la thérapeutique photodynamique.

Comme il sera démontré ci-après dans les exemples, ces composés ont permis de réaliser des études *in vitro* sur des cellules humaines cancéreuses connues pour être un bon modèle de xénogreffe, et il a été démontré que la thérapie photonique à un ou deux photons était efficace pour détruire de telles cellules.

Les références aux méthodes de traitement dans les paragraphes suivants de cette description doivent être interprétées comme des références aux composés, compositions pharmaceutiques et médicaments de la présente invention destinés à être utilisés dans une méthode de traitement du corps humain (ou animal) par thérapie (ou pour le diagnostic).

Selon la présente invention, les termes ci-dessous ont les significations suivantes : les termes mentionnés ici ayant des caractéristiques telles que par exemple C1-C18 peuvent également être utilisés avec des nombres inférieurs d'atomes de carbone tels que C1-C3 ou C1-C5. Si par exemple le terme C1-C5 est utilisé, il signifie que la chaîne hydrocarbonée correspondante peut comprendre de 1 à 5 atomes de carbone. Si par exemple le terme C3-C8 est utilisé, il signifie que la chaîne ou le cycle hydrocarboné correspondant peut comprendre de 3 à 8 atomes de carbone.

Un alkyl en C1-C18 selon l'invention est un alkyl comprenant 1, 2, 3, 4, 5, 6, 7, 8, 9, 10, 11, 12, 13, 14, 15, 16, 17 ou 18 atomes de carbone.

Par «alkyle», on entend un groupe aliphatique saturé, linéaire ou ramifié. On peut citer les exemples suivants : méthyle, éthyle, 1-propyle, 2-propyle, 1-butyle, 2-méthyl-1-propyle (également nommé i-Bu), 2-butyle (également nommé s-Bu), 2-méthyl-2-propyle (également nommé t-Bu), 1-pentyle (également nommé n-pentyle), 2-pentyle, 3-pentyle, 2-méthyl-2-butyle, 3-méthyl-2-butyle, 3-méthyl-1-butyle, 2-méthyl-1-butyle, 1-hexyl, 2-hexyl, 3-hexyl, 2-méthyl-2-pentyle, 3-méthyl-2-pentyle, 4-méthyl-2-pentyle, 3-méthyl-3-pentyle, 2-méthyl-3-pentyle, 2,3-diméthyl-2-butyle, 3,3-diméthyl-2-butyle, n-pentyle, n-hexyle, n-heptyle, H-octyle, n-nonyle, n-décyle, n-undécyle, n-dodécyle, n-tridécyle, n-tétradécyle, n-pentadécyle, n-hexadécyle, n-heptadécyle, n-octadécyle. L'alkyle préféré selon l'invention est le méthyle, l'éthyle, le 1-propyle, le 2-propyle, le 1-butyle, le 2-méthyl-1-propyle (également nommé i-Bu), 2-butyle (également nommé s-Bu), 2-méthyl-2-propyle (également nommé t-Bu), 1-pentyle (également nommé n-pentyle), 2-pentyle, 3-pentyle, le 2-méthyl-2-butyle, le 3-méthyl-2-butyle, le 3-méthyl-1-butyle, le 2-méthyl-1-butyle.

Le terme « hydroxyl » correspond à un groupe alkyle-OH, le groupe alkyle étant tel que défini ci-dessus.

Le terme « amino » correspond à une amine qui peut être, secondaire, tertiaire ou quaternaire. Par extension un « aminoalkyle » correspond à un alkyl substitué par une amine.

Le terme «alcoxy» correspond à un groupe-O-alkyle, le groupe alkyle étant tel que défini ci-dessus. Les exemples suivants peuvent être cités : méthoxy (i.e. C1 alcoxy), éthoxy (i.e. C2 alcoxy), propoxy (i.e. C3 alcoxy), isopropoxy (i.e. C4 alcoxy).

Le terme «atome d'halogène» correspond à un atome de fluor, de chlore, de brome ou d'iode. Le chlore est un atome d'halogène préféré dans le cadre de la présente invention.

Le terme «atome d'halogène» correspond à un atome de fluor, de chlore, de brome ou d'iode. Le chlore et le fluor sont des atomes d'halogènes préférés dans le cadre de la présente invention.

Le terme «aryle» utilisé ici signifie un groupe aromatique mono-ou poly-cyclique. Un exemple de groupe monocyclique peut être un phényle.

Les composés de formule (I) possèdent des insaturations et peuvent ainsi se présenter sous leur forme tautomère. La présente invention concerne donc également les composés de formule (I) sous leur forme tautomère.

Les composés de formule (I) peuvent se présenter sous la forme d'une base libre ou sous forme de sels d'addition avec des acides, qui font également partie de l'invention.

Ces sels peuvent être préparés avec des acides pharmaceutiquement acceptables, mais aussi des sels avec d'autres acides, utiles par exemple pour la purification ou pour l'isolement des composés de formule (I), font également partie de l'invention.

De manière avantageuse, l'invention concerne les composés susmentionnés où R1 et R2 sont, notamment indépendamment l'un de l'autre, des alkyles linéaires ou ramifiés saturés ou non, cyclique ou non, en C4-C10.

Des exemples d'alkyl linéaires saturés sont les groupes butyl, pentyl, hexyl, heptyl, octyl, nonyl et decyl.

Des exemples d'alkyl linéaires saturés ramifiés sont les groupes, isobutyle, sec-butyl, tert-butyl, isopentyl, néopentyl, tert-pentyl, 1-methyl isobutyl, 2,3-diméthylpentane, 2-méthylpropane, 2-méthylbutane, 2-méthylpentane, 2-éthylbutane, 3-méthylpropane, 3-méthylbutane, 3-méthylpentane

De manière plus avantageuse, l'invention concerne le composé susmentionné, où la forme protonée du composé de formule I est choisie parmi les composés suivants :
- le composé de formule la : 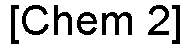 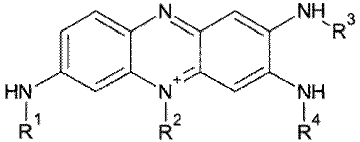 X⁻; (la),
- le composé de formule Ib : 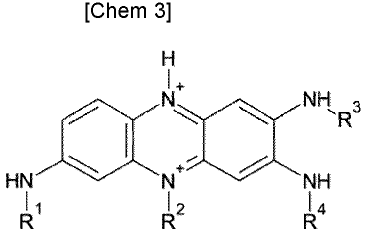 2X⁻; (Ib), et
- le composé de formle Ic : 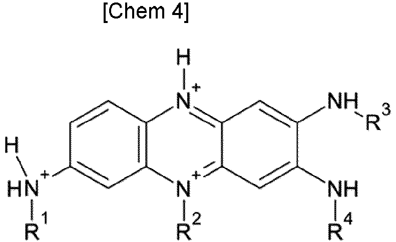 3X⁻ ; (Ic),
où X représente CI, Br, OH, F, I, BF₄ ou PF₆ ou trifluorométhanesulfate (Otf).

Les composés de formule la sont les composés les plus avantageux selon l'invention.

Aussi, avantageusement l'invention concerne un composé de formule la suivant : 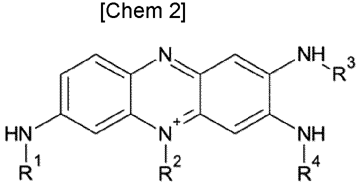 X⁻; (la),
où X représente CI, Br, OH, F, I, BF₄ ou PF₆ ou trifluorométhanesulfate , et où
indépendamment l'un de le l'autre, R1 et R2 sont un alkyle en C4-C10, linéaire ou ramifié, saturé ou non, cyclique ou non, et
indépendamment l'un de l'autre, R3 et 4 sont
   - ou H,
   - ou un alkyle en C1-C18, linéaire ou ramifié, saturé ou non, cyclique ou non, éventuellement substitué par un ou plusieurs groupe choisi parmi un groupe hydroxyl, un groupe amino, un groupe aminoalkyle, un groupe alkoxy en C1-C5, un alkyl en C1-C5, un peptide, un groupe pyridine, un groupe phosphine, un thiol, un C2 alcène, un groupe C2 alcyne et un halogène, or un radical benzyle éventuellement substitué par un ou plusieurs radicaux choisi parmi un groupe hydroxyl, un groupe amino, un groupe aminoalkyl, un groupe C1-C5 alkoxy, un groupe C1- C5 alkyl, et un atome d'halogène,
   - ou un groupe (hetero)aryle, éventuellement substitué par un ou plusieurs groupe choisi parmi un groupe hydroxyl, un groupe amino, un groupe amino alkyle, un groupe alkoxy en C1-C5, un alkyl en C1-C5, un peptide, un groupe pyridine, un groupe phosphine, un thiol, un C2 alcène, un groupe C2 alcyne et un halogène, or un radical benzyl éventuellement substitué par un ou plusieurs radicaux choisi parmi un groupe hydroxyl, un groupe amino, un groupe aminoalkyl, un groupe C1-C5 alkoxy, un groupe C1- C5 alkyl, et un halogène,
   - ou encore R3 et R4 sont l'un ou l'autre, ou les deux, un groupe fonctionnel carbonyle formant des fonctions amides incluant des peptides ou non,
ou un sel ou un solvate de celui-ci, ou une forme protonée de celui-ci.

Des composés selon l'invention qui sont avantageux, ou des composés de référence ne faisant pas partie de l'invention dont les R2 sont identifiés par un astérisque, sont les suivants :

**[Tableaux 1]**

| **Composé de formule** | **Où R1 est** | **Où R2 est** | **Où R3 est** | **Où R4 est** |
|---|---|---|---|---|
| 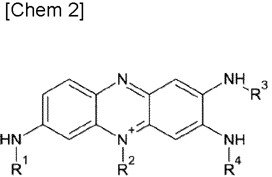 | octyl | octyl | octyl | H |
| | octyl | octyl | H | H |
| | octyl | octyl | *tert*-butyl | H |
| | *tert*-butyl | *tert*-butyl | *tert*-butyl | H |
| | nonyl | nonyl | nonyl | H |
| | nonyl | nonyl | H | H |
| | nonyl | nonyl | *tert*-butyl | H |
| | decyl | decyl | décyl | H |
| | decyl | decyl | H | H |
| | decyl | decyl | *tert*-butyl | H |
| | octyl | *H | octyl | H |
| | octyl | *H | H | H |
| | octyl | *H | *tert*-butyl | H |
| | *tert*-butyl | *H | *tert*-butyl | H |
| | nonyl | *H | nonyl | H |
| | nonyl | *H | H | H |
| | nonyl | *H | *tert*-butyl | H |
| | decyl | *H | décyl | H |
| | decyl | *H | H | H |
| | decyl | *H | *tert*-butyl | H |
| 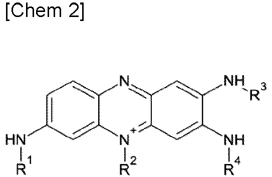 | octyl | octyl | octyl | C₁-C₁₈ |
| | octyl | octyl | H | C₁-C₁₈ |
| | octyl | octyl | *tert*-butyl | C₁-C₁₈ |
| | *tert*-butyl | *tert*-butyl | *tert*-butyl | C₁-C₁₈ |
| | nonyl | nonyl | nonyl | C₁-C₁₈ |
| | nonyl | nonyl | H | C₁-C₁₈ |
| | nonyl | nonyl | *tert*-butyl | C₁-C₁₈ |
| | decyl | decyl | décyl | C₁-C₁₈ |
| | decyl | decyl | H | C₁-C₁₈ |
| | decyl | decyl | *tert*-butyl | C₁-C₁₈ |
| | octyl | *H | octyl | C₁-C₁₈ |
| | octyl | *H | H | C₁-C₁₈ |
| | octyl | *H | *tert*-butyl | C₁-C₁₈ |
| | *tert*-butyl | *H | *tert*-butyl | C₁-C₁₈ |
| | nonyl | *H | nonyl | C₁-C₁₈ |
| | nonyl | *H | H | C₁-C₁₈ |
| | nonyl | *H | *tert*-butyl | C₁-C₁₈ |
| | decyl | *H | décyl | C₁-C₁₈ |
| | decyl | *H | H | C₁-C₁₈ |
| | decyl | *H | *tert*-butyl | C₁-C₁₈ |
| 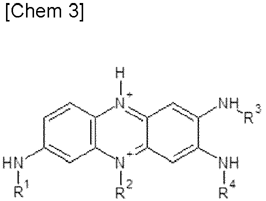 | octyl | octyl | octyl | H |
| | octyl | octyl | H | H |
| | octyl | octyl | *tert*-butyl | H |
| | *tert*-butyl | *tert*-butyl | *tert*-butyl | H |
| | nonyl | nonyl | nonyl | H |
| | nonyl | nonyl | H | H |
| | nonyl | nonyl | *tert*-butyl | H |
| | decyl | decyl | décyl | H |
| | decyl | decyl | H | H |
| | decyl | decyl | *tert*-butyl | H |
| | octyl | *H | octyl | H |
| | octyl | *H | H | H |
| | octyl | *H | *tert*-butyl | H |
| | *tert*-butyl | *H | *tert*-butyl | H |
| | nonyl | *H | nonyl | H |
| | nonyl | *H | H | H |
| | nonyl | *H | *tert*-butyl | H |
| | decyl | *H | décyl | H |
| | decyl | *H | H | H |
| | decyl | *H | *tert*-butyl | H |
| 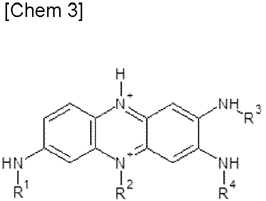 | octyl | octyl | octyl | C₁-C₁₈ |
| | octyl | octyl | H | C₁-C₁₈ |
| | octyl | octyl | *tert*-butyl | C₁-C₁₈ |
| | *tert*-butyl | *tert*-butyl | *tert*-butyl | C₁-C₁₈ |
| | nonyl | nonyl | nonyl | C₁-C₁₈ |
| | nonyl | nonyl | H | C₁-C₁₈ |
| | nonyl | nonyl | *tert*-butyl | C₁-C₁₈ |
| | decyl | decyl | décyl | C₁-C₁₈ |
| | decyl | decyl | H | C₁-C₁₈ |
| | decyl | decyl | *tert*-butyl | C₁-C₁₈ |
| | octyl | *H | octyl | C₁-C₁₈ |
| | octyl | *H | H | C₁-C₁₈ |
| | octyl | *H | *tert*-butyl | C₁-C₁₈ |
| | *tert*-butyl | *H | *tert*-butyl | C₁-C₁₈ |
| | nonyl | *H | nonyl | C₁-C₁₈ |
| | nonyl | *H | H | C₁-C₁₈ |
| | nonyl | *H | *tert*-butyl | C₁-C₁₈ |
| | decyl | *H | décyl | C₁-C₁₈ |
| | decyl | *H | H | C₁-C₁₈ |
| | decyl | *H | *tert*-butyl | C₁-C₁₈ |
| 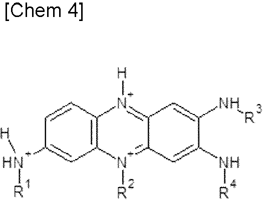 | octyl | octyl | octyl | H |
| | octyl | octyl | H | H |
| | octyl | octyl | tert-butyl | H |
| | *tert*-butyl | *tert*-butyl | *tert*-butyl | H |
| | nonyl | nonyl | nonyl | H |
| | nonyl | nonyl | H | H |
| | nonyl | nonyl | *tert*-butyl | H |
| | decyl | decyl | décyl | H |
| | decyl | decyl | H | H |
| | decyl | decyl | *tert*-butyl | H |
| | octyl | *H | octyl | H |
| | octyl | *H | H | H |
| | octyl | *H | *tert*-butyl | H |
| | *tert*-butyl | *H | *tert*-butyl | H |
| | nonyl | *H | nonyl | H |
| | nonyl | *H | H | H |
| | nonyl | *H | *tert*-butyl | H |
| | decyl | *H | décyl | H |
| | decyl | *H | H | H |
| | decyl | *H | *tert*-butyl | H |
| 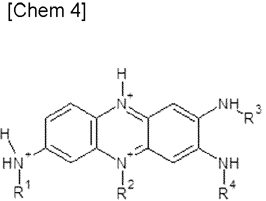 | octyl | octyl | octyl | C₁-C₁₈ |
| | octyl | octyl | H | C₁-C₁₈ |
| | octyl | octyl | *tert*-butyl | C₁-C₁₈ |
| | *tert*-butyl | *tert*-butyl | *tert*-butyl | C₁-C₁₈ |
| | nonyl | nonyl | nonyl | C₁-C₁₈ |
| | nonyl | nonyl | H | C₁-C₁₈ |
| | nonyl | nonyl | *tert*-butyl | C₁-C₁₈ |
| | decyl | decyl | décyl | C₁-C₁₈ |
| | decyl | decyl | H | C₁-C₁₈ |
| | decyl | decyl | *tert*-butyl | C₁-C₁₈ |
| | octyl | *H | octyl | C₁-C₁₈ |
| | octyl | *H | H | C₁-C₁₈ |
| | octyl | *H | *tert*-butyl | C₁-C₁₈ |
| | *tert*-butyl | *H | *tert*-butyl | C₁-C₁₈ |
| | nonyl | *H | nonyl | C₁-C₁₈ |
| | nonyl | *H | H | C₁-C₁₈ |
| | nonyl | *H | *tert*-butyl | C₁-C₁₈ |
| | decyl | *H | décyl | C₁-C₁₈ |
| | decyl | *H | H | C₁-C₁₈ |
| | decyl | *H | *tert*-butyl | C₁-C₁₈ |
| 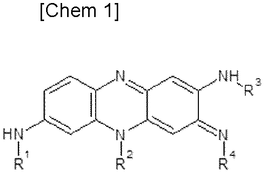 | octyl | octyl | octyl | H |
| | octyl | octyl | H | H |
| | octyl | octyl | *tert*-butyl | H |
| | *tert*-butyl | *tert*-butyl | *tert*-butyl | H |
| | nonyl | nonyl | nonyl | H |
| | nonyl | nonyl | H | H |
| | nonyl | nonyl | *tert*-butyl | H |
| | decyl | decyl | décyl | H |
| | decyl | decyl | H | H |
| | decyl | decyl | *tert*-butyl | H |
| | octyl | *H | octyl | H |
| | octyl | *H | H | H |
| | octyl | *H | *tert*-butyl | H |
| | *tert*-butyl | *H | *tert*-butyl | H |
| | nonyl | *H | nonyl | H |
| | nonyl | *H | H | H |
| | nonyl | *H | *tert*-butyl | H |
| | decyl | *H | décyl | H |
| | decyl | *H | H | H |
| | decyl | *H | *tert*-butyl | H |
| 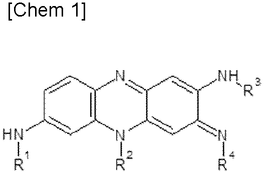 | octyl | octyl | octyl | C₁-C₁₈ |
| | octyl | octyl | H | C₁-C₁₈ |
| | octyl | octyl | *tert*-butyl | C₁-C₁₈ |
| | *tert*-butyl | *tert*-butyl | *tert*-butyl | C₁-C₁₈ |
| | nonyl | nonyl | nonyl | C₁-C₁₈ |
| | nonyl | nonyl | H | C₁-C₁₈ |
| | nonyl | nonyl | *tert*-butyl | C₁-C₁₈ |
| | decyl | decyl | décyl | C₁-C₁₈ |
| | decyl | decyl | H | C₁-C₁₈ |
| | decyl | decyl | *tert*-butyl | C₁-C₁₈ |
| | octyl | *H | octyl | C₁-C₁₈ |
| | octyl | *H | H | C₁-C₁₈ |
| | octyl | *H | *tert*-butyl | C₁-C₁₈ |
| | *tert*-butyl | *H | *tert*-butyl | C₁-C₁₈ |
| | nonyl | *H | nonyl | C₁-C₁₈ |
| | nonyl | *H | H | C₁-C₁₈ |
| | nonyl | *H | *tert*-butyl | C₁-C₁₈ |
| | decyl | *H | décyl | C₁-C₁₈ |
| | decyl | *H | H | C₁-C₁₈ |
| | decyl | *H | *tert*-butyl | C₁-C₁₈ |

De manière plus avantageuse, l'invention concerne le composé susmentionné, ledit composé ayant la formule II, III ou IV suivante : 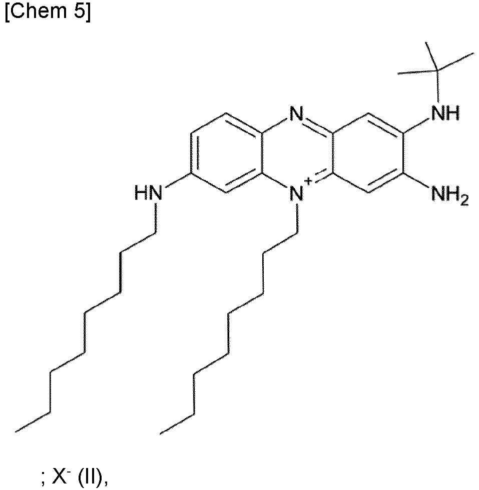 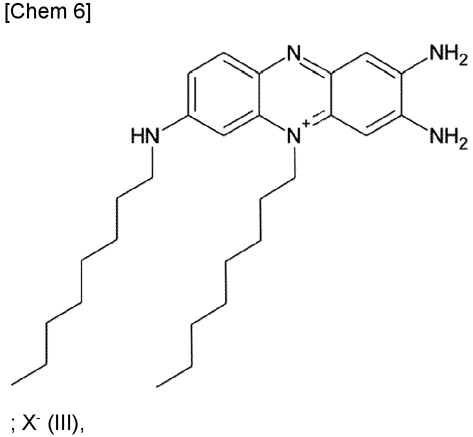 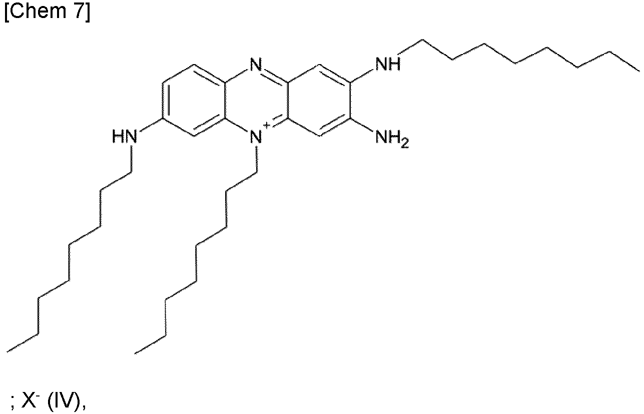 où X représente CI, Br, OH, F ou I.

L'invention concerne en outre une composition pharmaceutique comprenant à titre de substance active un composé tel que défini ci-dessus, en association avec un véhicule pharmaceutiquement acceptable.

L'invention concerne un composé susmentionné, pour son utilisation en tant que médicament.

Selon la présente invention, un dérivé pharmaceutiquement acceptable comprend, mais n'est pas limité à, des sels pharmaceutiquement acceptables, des esters, des sels de tels esters, ou tout autre produit d'addition ou dérivé qui, lors de l'administration à un patient dans le besoin, est capable de fournir, directement ou indirectement, un composé tel que décrit ci-dessus, ou un métabolite ou un résidu de celui-ci, par exemple, un promédicament.

Les composés selon l'invention sont également vectorisable, en particulier via R4, en greffant des ligands naturels spécifiquement reconnus par les cellules cancéreuses. Ces biomolécules peuvent être des stéroïdes, des sucres (glucose et dérivés), des amines, des acides aminés ou des peptides.

Par " véhicule pharmaceutiquement acceptable ", tel que mentionné ci-dessus, on entend un ou plusieurs solvants, diluants, ou un autre véhicule liquide, des auxiliaires de dispersion ou de suspension, des agents tensioactifs, des agents isotoniques, des agents épaississants ou émulsifiants, des conservateurs, des liants solides, des lubrifiants et analogues, selon la forme posologique particulière souhaitée. Sauf dans la mesure où un support classique est incompatible avec les composés de l'invention, par exemple par la production d'un quelconque effet biologique indésirable ou d'une interaction différente d'une manière délétère avec n'importe quel(s) autre(s) composant(s) de la composition pharmaceutique susmentionnée, l'utilisation de tout support connu est envisagée dans le cadre de la présente invention.

Certains exemples de matériaux qui peuvent servir de supports pharmaceutiquement acceptables sont, sans limitation, des sucres tels que le lactose, le glucose et le saccharose; les amidons tels que l'amidon de maïs et l'amidon de pomme de terre; la cellulose et ses dérivés tels que la carboxyméthylcellulose de sodium, la cellulose d'éthyle et l'acétate de cellulose; le tragacanthe en poudre; le malt; la gélatine; le talc; les excipients tels que le beurre de cacao et les cires de suppositoire; les huiles telles que l'huile d'arachide, l'huile de coton; l'huile de carthame; l'huile de sésame; l'huile d'olive; l'huile de maïs et l'huile de soja; les glycols; un tel propylène glycol; des esters tels que l'oléate d'éthyle et le laurate d'éthyle; de l'agar; des agents tampons tels que de l'hydroxyde de magnésium et de l'hydroxyde d'aluminium; de l'acide alginique; de l'eau exempte de pyrogène; une solution saline isotonique; une solution de Ringer; de l'alcool éthylique et des solutions tampons de phosphate, ainsi que d'autres lubrifiants compatibles non toxiques tels que le laurylsulfate de sodium et le stéarate de magnésium, ainsi que des agents colorants, des agents de libération, des agents de revêtement, des édulcorants, des agents aromatisants et parfumantes.

Des conservateurs et des antioxydants peuvent également être présents dans la composition, en fonction du jugement du formulateur.

Un composé selon l'invention est de préférence formulé sous forme d'unité posologique pour faciliter l'administration et l'uniformité de dosage. On comprend toutefois que l'usage quotidien total des composés et des compositions de la présente invention sera décidé par le médecin traitant dans le cadre de l'évaluation médicale. Le niveau de dose thérapeutiquement efficace spécifique pour n'importe quel patient ou organisme particulier dépendra d'une variété de facteurs comprenant le trouble en cours de traitement et la gravité du trouble; l'activité du composé spécifique employé; la composition spécifique utilisée; l'âge, le poids corporel, la santé générale, le sexe et le régime du patient; le temps d'administration, la voie d'administration et la vitesse d'excrétion du composé spécifique employé; la durée du traitement; Des médicaments utilisés en combinaison ou en coïncidence avec le composé spécifique employé; et des facteurs similaires bien connus dans les arts médicaux.

En outre, après formulation avec un excipient ou véhicule pharmaceutiquement acceptable approprié dans un dosage souhaité, les compositions pharmaceutiques selon l'invention peuvent être administrées à des êtres humains et à d'autres animaux par voie orale, rectale, parentérale, intracistemale, intravaginale, par voie intrapéritonéale, par voie dermale (par exemple par des poudres, des pommades ou des gouttes), par voie buccale, sous la forme d'une pulvérisation orale ou nasale, ou similaire en fonction de la gravité de la maladie ou du cancer en cours de traitement.

Les composés actifs selon l'invention peuvent également être sous forme microencapsulée, éventuellement avec un ou plusieurs excipients tels que notés ci-dessus.

Il est également apprécié que les composés et les compositions pharmaceutiques de la présente invention puissent être utilisés dans des polythérapies, à savoir, les composés et les compositions pharmaceutiques peuvent être administrés simultanément avec, avant, ou après, un ou plusieurs autres agents thérapeutiques ou procédures médicales souhaités.

De manière avantageuse, le composé selon l'invention possède des propriétés colorantes, et peut être utilisé en tant que colorant. De tels colorants sont notamment de couleur rouge, et sont capables de colorer (ou pigmenter) des fibres végétales ou animales.

**Du** fait de leurs propriétés fluorescentes, les composés selon l'invention peuvent également être utilisés en tant que « colorants » fluorescents, notamment pour le marquage de molécules biologiques ou d'organites cellulaires.

Dans un autre aspect, l'invention concerne un composé tel que défini précédemment, pour son utilisation dans le cadre du traitement de pathologies par thérapie photodynamique. En particulier, les pathologies pouvant être traitées par le composé selon l'invention, à l'aide d'une thérapie photodynamique, sont les tumeurs (solides ou hématopoïétiques) et les kératoses.

Une fois un cancer diagnostiqué, différentes méthodes sont à la disposition des praticiens pour traiter la maladie. Ces différentes techniques peuvent être combinées et le choix du traitement dépend du type de cancer et du stade auquel il a été découvert :
- la chirurgie est classiquement utilisée pour enlever la tumeur primaire et permet la guérison d'un grand nombre de cancers précoces. Elle est aujourd'hui la méthode la plus efficace pour les petits foyers tumoraux sans métastase. Cependant, l'élimination de l'intégralité des cellules cancéreuses et la prévention de leur dissémination lors de l'opération chirurgicale peuvent être difficiles.
- la radiothérapie, basée sur l'action de rayonnements ionisants (X, α, β- ou y), est utilisée pour traiter les tumeurs mais pose le problème de la toxicité des rayonnements ionisants sur les tissus sains environnants.
- la chimiothérapie consiste à traiter le cancer au moyen de médicaments qui détruisent les cellules cancéreuses et les empêchent de se multiplier. Il existe de nombreux médicaments différents, le choix du traitement dépendant du type de cancer. Cependant, ils ne sont pas encore assez spécifiques puisqu'ils ne font pas encore la différence entre les cellules saines et les cellules cancéreuses, entrainant ainsi de nombreux effets secondaires.
- la thérapie photodynamique (PDT) consiste à mettre en contact un tissu pathologique avec une molécule photo activable (nommée photo-sensibilisateur), puis à photo-activer la molécule par la lumière de façon à produire de l'oxygène singlet, très toxiques, qui va localement détruire la lésion cancéreuse. L'avantage majeur de la PDT est sa sélectivité. En effet, la lumière utilisée, seule n'est pas nocive, le photo-sensibilisateur sans lumière n'est pas toxique. Pour induire la réaction, il faut une action conjointe de la lumière, du photo-sensibilisateur et de l'oxygène. Ainsi, en optimisant la concentration du photo-sensibilisateur et la dose de lumière (puissance du laser), il est possible de détruire sélectivement des cellules.

Sur le plan thérapeutique, les traitements conventionnels ont une sélectivité imparfaite vis-à-vis des cellules tumorales. Une des raisons est que pendant longtemps les scientifiques ont privilégie la course à l'IC₅₀ (concentration de produit nécessaire pour tuer 50% d'une population de cellules) plutôt que la recherche de spécificité. Ils entrainent des effets secondaires, parfois sévères, qui limitent les doses auxquelles ils peuvent être administres.

La cancérologie clinique réclame donc le développement conjoint de nouvelles méthodes diagnostiques plus sensibles et plus performantes, ainsi que de nouvelles thérapeutiques plus efficaces, mieux tolérées mais aussi mieux comprises.

Sur la base de ce besoin, les inventeurs ont mis à profit les propriétés peu toxiques des composés selon l'invention, et leur capacité à produire de l'oxygène singulet sous excitation lumineuse afin de proposer une thérapie photodynamique.

Les inventeurs proposent ainsi une thérapie photodynamique utilisant les composés susmentionnés soit :
- A un photon (λ_{irrad}. = 514 nm), où les cibles visées sont les tumeurs de surface (le mélanome, la vessie, l'œsophage et des bronches) car la longueur d'onde d'excitation est dans le vert. Bien que l'excitation dans le rouge lointain (652 nm) ou le proche infrarouge (760 nm) soit privilégiée, des études ont démontré que pour des cas particuliers, l'excitation par une lumière verte était moins toxique et plus efficace que l'excitation par une lumière rouge. Par exemple, dans une étude réalisée avec le Photofrin, sur des xénogreffes de mésothéliome humain chez des souris *nude,* il a été démontré qu'une thérapie photodynamique réalisée avec une lumière de 514 nm pouvait induire des effets au niveau de la tumeur, similaires à ceux obtenus avec une excitation a 630 nm, avec une diminution des lésions des tissus normaux. La lumière verte prévient les lésions profondes des tissus, réduisant ainsi le risque de perforation. Depuis, de nombreuses études sur des cellules en culture ou bien sur des animaux de laboratoire l'ont confirmé.
- A deux photons (λ_{irrad.} = 810 nm), les cibles visées sont les tumeurs plus profondes (cancer du sein, de la prostate, rétinoblastome) car l'excitation de la molécule avec un laser dans le rouge lointain ou le proche infra-rouge permet une pénétration plus profonde des tissus (zone de transparence biologique).

De manière avantageuse, l'utilisation médicale selon l'invention permet le traitement de pathologies, notamment le traitement de tumeurs, par thérapie photodynamique, ledit traitement comprenant une étape d'administration à des individus dans le besoin une dose efficace de composé tel que défini ci-dessus, et une exposition à un faisceau lumineux ayant une longueur d'onde variant de 450 à 850 nm.

De manière avantageuse, l'utilisation médicale susmentionnée permet le traitement de pathologies, notamment le traitement de tumeurs, par thérapie photodynamique à un photon, ledit traitement comprenant une étape d'administration à des individus dans le besoin une dose efficace de composé tel que défini ci-dessus, et une exposition à un faisceau lumineux ayant une longueur d'onde variant de 450 à 550 nm.

Par « longueur d'onde variant de 450 à 550 nm » on entend dans l'invention une longueur d'onde de 450nm, 451nm, 452nm, 453nm, 454nm, 455nm, 456nm, 457nm, 458nm, 459nm, 460nm, 461nm, 462nm, 463nm, 464nm, 465nm, 466nm, 467nm, 468nm, 469nm, 470nm, 471nm, 472nm, 473nm, 474nm, 475nm, 476nm, 477nm, 478nm, 479nm, 480nm, 481nm, 482nm, 483nm, 484nm, 485nm, 486nm, 487nm, 488nm, 489nm, 490nm, 491nm, 492nm, 493nm, 494nm, 495nm, 496nm, 497nm, 498nm, 499nm, 500nm, 501nm, 502nm, 503nm, 504nm, 505nm, 506nm, 507nm, 508nm, 509nm, 510nm, 511nm, 512nm, 513nm, 514nm, 515nm, 516nm, 517nm, 518nm, 519nm, 520nm, 521nm, 522nm, 523nm, 524nm, 525nm, 526nm, 527nm, 528nm, 529nm, 530nm, 531nm, 532nm, 533nm, 534nm, 535nm, 536nm, 537nm, 538nm, 539nm, 540nm, 541nm, 542nm, 543nm, 544nm, 545nm, 546nm, 547nm, 548nm, 549nm ou 550nm.

De manière avantageuse, l'utilisation susmentionnée permet le traitement de pathologies, notamment le traitement de tumeurs, par thérapie photodynamique à deux photons, ledit traitement comprenant une étape d'administration à des individus dans le besoin une dose efficace de composé tel que défini ci-dessus, et une exposition à un faisceau lumineux ayant une longueur d'onde variant de 750 à 850 nm.

Par « longueur d'onde variant de 750 à 850 nm » on entend dans l'invention une longueur d'onde de 750nm, 751nm, 752nm, 753nm, 754nm, 755nm, 756nm, 757nm, 758nm, 759nm, 760nm, 761nm, 762nm, 763nm, 764nm, 765nm, 766nm, 767nm, 768nm, 769nm, 770nm, 771nm, 772nm, 773nm, 774nm, 775nm, 776nm, 777nm, 778nm, 779nm, 780nm, 781nm, 782nm, 783nm, 784nm, 785nm, 786nm, 787nm, 788nm, 789nm, 790nm, 791nm, 792nm, 793nm, 794nm, 795nm, 796nm, 797nm, 798nm, 799nm, 500nm, 501nm, 502nm, 503nm, 504nm, 505nm, 506nm, 507nm, 508nm, 509nm, 510nm, 511nm, 512nm, 513nm, 514nm, 515nm, 516nm, 517nm, 518nm, 519nm, 520nm, 521nm, 522nm, 523nm, 524nm, 525nm, 526nm, 527nm, 528nm, 529nm, 530nm, 531nm, 532nm, 533nm, 534nm, 535nm, 536nm, 537nm, 538nm, 539nm, 540nm, 541nm, 542nm, 543nm, 544nm, 545nm, 546nm, 847nm, 848nm, 849nm ou 850nm.

Dans l'un quelconque des traitements susmentionnés, il est avantageux d'administrer un composé selon l'invention à une dose variant de 2 nmol.L⁻¹ (ou nM) à 1 µmol.L⁻¹ (ou 1000 nmol.L⁻¹), notamment de 2 nmol.L⁻¹ (ou nM) à 1 µmol.L⁻¹ par kg pour une administration *per os.* Ainsi pour un individu de 70 kg en moyenne, la dose administrée sera de 140 nM à 70 µM. Dans le cadre d'une administration ciblée, (par injection directement dans la tumeur par exemple), la dose sera directement celle décrite plus haut sans le coefficient multiplicateur de masse en kg.

On entend par «une dose variant de 2 nmol.L⁻¹ à 1 µmol.L⁻¹ » une dose de 2 nmol.L⁻¹, 3 nmol.L⁻¹, 4 nmol.L⁻¹, 5 nmol.L⁻¹, 6 nmol.L⁻¹, 7 nmol.L⁻¹, 8 nmol.L⁻¹, 9 nmol.L⁻¹, 10 nmol.L⁻¹, 15 nmol.L⁻¹, 20 nmol.L⁻¹, 25 nmol.L⁻¹, 30 nmol.L⁻¹, 35 nmol.L⁻¹, 40 nmol.L⁻¹, 45 nmol.L⁻¹, 50 nmol.L⁻¹, 55 nmol.L⁻¹, 60 nmol.L⁻¹, 65 nmol.L⁻¹, 70 nmol.L⁻¹, 75 nmol.L⁻¹, 80 nmol.L⁻¹, 85 nmol.L⁻¹, 90 nmol.L⁻¹, 95 nmol.L⁻¹, 100 nmol.L⁻¹, 105 nmol.L⁻¹, 110 nmol.L⁻¹, 115 nmol.L⁻¹, 120 nmol.L⁻¹, 125 nmol.L⁻¹, 130 nmol.L⁻¹, 135 nmol.L⁻¹, 140 nmol.L⁻¹, 145 nmol.L⁻¹, 150 nmol.L⁻¹, 155 nmol.L⁻¹, 160 nmol.L⁻¹, 165 nmol.L⁻¹, 170 nmol.L⁻¹, 175 nmol.L⁻¹, 180 nmol.L⁻¹, 185 nmol.L⁻¹, 190 nmol.L⁻¹, 195 nmol.L⁻¹, 200 nmol.L⁻¹, 205 nmol.L⁻¹, 210 nmol.L⁻¹, 215 nmol.L⁻¹, 220 nmol.L⁻¹, 225 nmol.L⁻¹, 230 nmol.L⁻¹, 235 nmol.L⁻¹, 240 nmol.L⁻¹, 245 nmol.L⁻¹, 250 nmol.L⁻¹, 255 nmol.L⁻¹, 260 nmol.L⁻¹, 265 nmol.L⁻¹, 270 nmol.L⁻¹, 275 nmol.L⁻¹, 280 nmol.L⁻¹, 285 nmol.L⁻¹, 290 nmol.L⁻¹, 295 nmol.L⁻¹, 300 nmol.L⁻¹, 305 nmol.L⁻¹, 310 nmol.L⁻¹, 315 nmol.L⁻¹, 320 nmol.L⁻¹, 325 nmol.L⁻¹, 330 nmol.L⁻¹, 335 nmol.L⁻¹, 340 nmol.L⁻¹, 345 nmol.L⁻¹, 350 nmol.L⁻¹, 355 nmol.L⁻¹, 360 nmol.L⁻¹, 365 nmol.L⁻¹, 370 nmol.L⁻¹, 375 nmol.L⁻¹, 380 nmol.L⁻¹, 385 nmol.L⁻¹, 390 nmol.L⁻¹, 395 nmol.L⁻¹, 400 nmol.L⁻¹, 405 nmol.L⁻¹, 410 nmol.L⁻¹, 415 nmol.L⁻¹, 420 nmol.L⁻¹, 425 nmol.L⁻¹, 430 nmol.L⁻¹, 435 nmol.L⁻¹, 440 nmol.L⁻¹, 445 nmol.L⁻¹, 450 nmol.L⁻¹, 455 nmol.L⁻¹, 460 nmol.L⁻¹, 465 nmol.L⁻¹, 470 nmol.L⁻¹, 475 nmol.L⁻¹, 480 nmol.L⁻¹, 485 nmol.L⁻¹, 490 nmol.L⁻¹, 495 nmol.L⁻¹, 500 nmol.L⁻¹, 525 nmol.L⁻¹, 550 nmol.L⁻¹, 575 nmol.L⁻¹, 600 nmol.L⁻¹, 625 nmol.L⁻¹, 650 nmol.L⁻¹, 675 nmol.L⁻¹, 700 nmol.L⁻¹, 725 nmol.L⁻¹, 750 nmol.L⁻¹, 775 nmol.L⁻¹, 800 nmol.L⁻¹, 825 nmol.L⁻¹, 850 nmol.L⁻¹, 875 nmol.L⁻¹, 900 nmol.L⁻¹, 925 nmol.L⁻¹, 950 nmol.L⁻¹, 975 nmol.L⁻¹ ou 1000 nmol.L⁻¹.

La thérapie photodynamique (photodynamic therapy, PDT) est utilisée depuis de nombreuses années en dermatologie. Son principe théorique repose sur l'utilisation d'une molécule inoffensive, s'accumulant de manière préférentielle dans les cellules à traiter. Cette molécule est transformée en une molécule cytotoxique après excitation lumineuse. La spécificité du traitement vient d'une part de la pharmacocinétique de la molécule (diffusion, absorption et métabolisation cellulaire) et d'autre part, par la physique du flux lumineux.

La thérapie photodynamique antitumorale est donc basée sur l'association de molécules photosensibilisantes (Ps) capables de se concentrer dans les cellules tumorales, et d'une lumière focalisée de longueur d'onde appropriée (dépendante du Ps). Ce sera la combinaison de ces deux facteurs qui permettra de cibler spécifiquement les tissus tumoraux et de les détruire. Cette méthode présente tout de même un inconvénient de taille : seuls les cancers accessibles à la lumière peuvent être traités (la lumière rouge par exemple n'a qu'une pénétration d'environ 1 cm dans les tissus vivants).

L'action de la lumière (à une longueur d'onde soigneusement sélectionnée) sur le sensibilisateur va entraîner la formation d'oxygène singulet ¹O₂ (de courte durée de vie de l'ordre de 0,01 à 0,004 µs), molécule très réactive vis-à-vis des composants cellulaires et donc très toxique. Le photosensibilisateur est injecté par voie intraveineuse ; Celui-ci va se concentrer plus ou moins sélectivement dans le tissu tumoral dont l'irradiation par une lumière laser de longueur d'onde appropriée au colorant utilisé conduit à la nécrose ou l'apoptose des cellules cancéreuse.

Les inventeurs ont fait la constatation surprenante que les composés selon l'invention, une fois activés à des longues d'onde spécifiques, sont capables de produire des espèces oxygénées réactives sous forme d'oxygène singulet avec un rendement quantique Φ_{Δ} de l'ordre de 0,1. Ce faible rendement est attendu - puisque la grande majorité des photons absorbés est convertie en lumière, 76%.

Mais de manière surprenante, les inventeurs ont remarqué que ce rendement est largement suffisant pour conduire *in vitro* à la destruction de 98% des cellules tumorales à des concentrations très faibles de l'ordre de 100 nM.

L'invention concerne en outre un composé tel que défini précédemment, pour son utilisation dans le cadre du diagnostic de pathologies, notamment de cancers.

L'imagerie par fluorescence est l'une des techniques les plus puissantes pour observer les processus intracellulaires dynamiques dans les cellules vivantes. L'accès à de nouvelles sondes fluorescentes adaptables est d'une importance majeure car seules très peu de biomolécules peuvent actuellement être visualisées en raison de limitations inhérentes à la structure des sondes utilisées à ce jour.

L'imagerie proposée, ou l'utilisation des composés selon l'invention, repose sur la fluorescence classique à 1 photon, ou à deux photons, comme exposé ci-dessus.

Une méthode d'imagerie par fluorescence *in vivo,* peut comprendre une étape d'administration à des individus dans le besoin un dose efficace de composé tel que défini ci-dessus, et une exposition à un faisceau lumineux ayant une longueur d'onde variant de 450 à 850 nm, et une étape de détection par des moyens appropriés des cellules, tissus ou organites cellules fluorescents.

Une méthode d'imagerie par fluorescence *in vivo,* peut comprendre une étape d'administration à des individus dans le besoin une dose efficace de composé tel que défini ci-dessus, et une exposition à un faisceau lumineux ayant une longueur d'onde variant de 450 à 550 nm, et une étape de détection par des moyens appropriés de détection de fluorescence à 1 photon des cellules, tissus ou organites cellules fluorescents.

Une méthode d'imagerie par fluorescence *in vivo,* peut comprendre une étape d'administration à des individus dans le besoin une dose efficace de composé tel que défini ci-dessus, et une exposition à un faisceau lumineux ayant une longueur d'onde variant de 750 à 850 nm, et une étape de détection par des moyens appropriés de détection de fluorescence à 2 photons des cellules, tissus ou organites cellules fluorescents.

L'invention concerne également l'utilisation d'un composé susmentionné, pour la visualisation, *in vitro* ou *ex vivo,* de cellules vivantes, d'organites du cytoplasme (mitochondrie, réticulum endoplasmique, appareil de Golgi, vésicules, noyau, etc...) ou de tissus, par microscopie à fluorescence à un ou deux photons, ledit composé étant notamment utilisé à une concentration variant de 2 à 500 nmol. L⁻¹.

Une méthode, notamment *in vitro,* de visualisation de cellules vivantes, d'organites du cytoplasme (mitochondrie, réticulum endoplasmique, appareil de Golgi, vésicules, noyau, etc...) ou de tissus, par microscopie à fluorescence, peut comprendre :
- une étape de mise en contact cellules vivantes, d'organites du cytoplasme ou de tissus, préalablement prélevés sur un individu ou un animal, avec un composé susmentionné, à une concentration de 2 à 500 nmol. L⁻¹.
- une étape d'exposition à un faisceau lumineux ayant une longueur d'onde variant de 450 à 850 nm, et
- une étape de détection par des moyens appropriés de détection de fluorescence à 1 ou 2 photons les cellules, les tissus ou les organites fluorescents.

L'invention concerne par ailleurs une méthode *in vitro,* d'éradication de cellules comprenant une étape d'exposition, à l'aide d'une source lumineuse émettant un ou deux photons, de cellules traitées avec un composé selon l'une quelconque des revendications 1 à 4, ledit composé étant utilisé à une concentration variant de 1 à 1000 nmol. L⁻¹.

L'invention concerne donc l'utilisation d'un composé susmentionné, pour l'éradication *in vitro* de cellules.

De manière avantageuse l'invention concerne le diagnostic, notamment in vitro, d'une pathologie impliquant une dérégulation de l'expression ou de l'activité d'une ou plusieurs peptidases, amidases ou les deux, à partir d'un échantillon biologique issu d'individus atteint par ladite pathologie comprenant :
- une étape de mise en contact dudit échantillon biologique avec un composé tel que défini dans l'une quelconque des revendications 1 à 2, ou R4 est un groupe fonctionnel inhibant les propriétés fluorescentes dudit composé,
- une étape de détection de la fluorescence après exposition à un faisceau lumineux ayant une longueur d'onde variant de 450 à 850 nm.

Les inventeurs ont fait la constatation surprenante que certains groupes R4, notamment (Carbonyl de type C(O-alkyle ou C(O)-aryle) modifie de façon drastique les propriétés de fluorescence des composés selon l'invention. Par contre, une fois que ces groupes sont clivés, par la rupture de la fonction amide, les composés recouvrent leurs capacités de fluorescence initiale.

Ainsi, les composés de l'invention où R4 est un groupe impactant fortement la fluorescence peuvent servir de sonde diagnostic pour détecter au sein de cellules une activité anormale de peptidase ou d'amidase, dérégulation qui est corrélée à une pathologie.

Dès lors si la cellule est saine, aucune fluorescence ne sera émise après excitation, ou une fluorescence à un certain niveau F0 sera mesurable. Si par contre la cellule est une cellule ayant une activité peptidade/amidase augmentée par rapport à la cellule saine, ou simplement se met à exprimer la peptidade/amidase qui n'est pas exprimée dans la cellule saine, le groupe R4 sera clivé, ou sera plus clivé, et une différence de fluorescence sera observée.

De la même manière, si la cellule pathologique à une diminution de l'activité peptidade/amidase par rapport à la cellule saine, la fluorescence dans les cellules pathologiques sera plus faible, voire disparaitra.

L'invention sera mieux comprise à la lumière des figures ci-après décrites et des exemples qui suivent.

### BREVE DESCRIPTION DES DESSINS

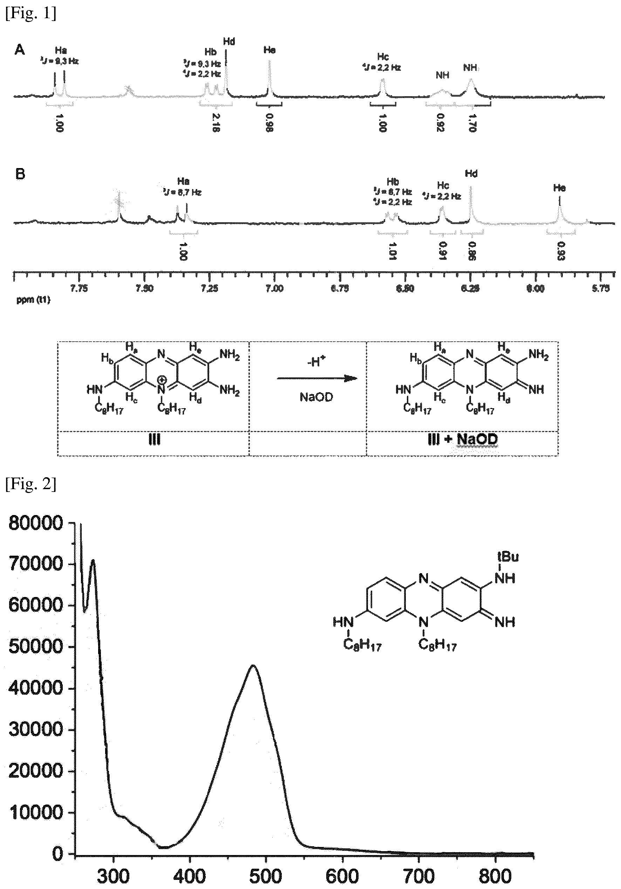

[Fig. 1] La figure 1 Spectres RMN 1H de I dans le MeCN-d3 : sans ajout de NaOD (A), avec ajout de NaOD (B), (la fenêtre entre δ = 5,7 ppm et 0 ppm a été omise pour des raisons de clarté). : Le tableau indique la forme protonée et la forme non protonée en présence de NaOD. L'axe x représente les valeurs en ppm.
[Fig. 2] La figure 2 représente le spectre d'absorption ε (M⁻¹cm⁻¹) en fonction de la longueur d'onde λ (en nm) du composé représenté.

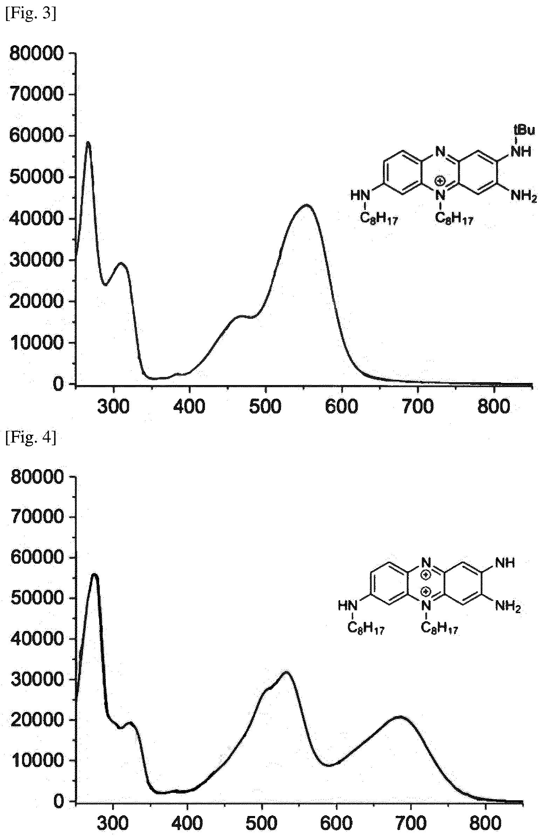

[Fig. 3] La figure 3 représente le spectre d'absorption ε (M⁻¹cm⁻¹) en fonction de la longueur d'onde λ (en nm) du composé représenté.
[Fig. 4] La figure 4 représente le spectre d'absorption ε (M⁻¹cm⁻¹) en fonction de la longueur d'onde λ (en nm) du composé représenté.

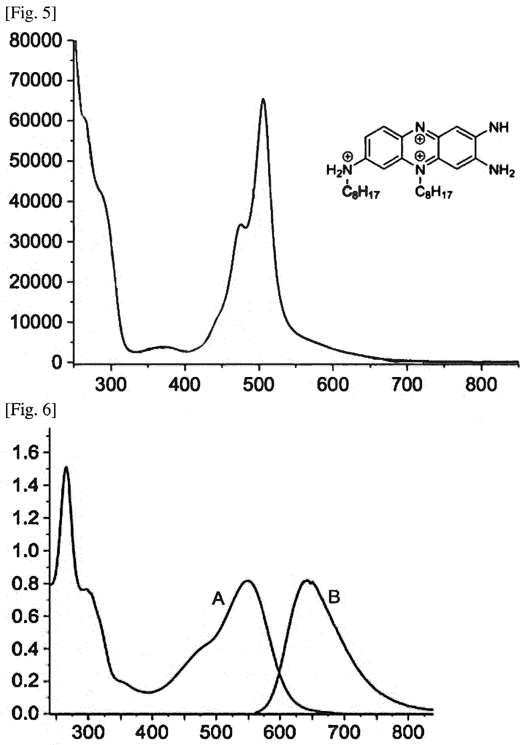

[Fig. 5] La figure 5 représente le spectre d'absorption ε (M⁻¹cm⁻¹) en fonction de la longueur d'onde λ (en nm) du composé représenté.
[Fig. 6] La figure 6 représente le spectre d'absorption (A) en fonction de la longueur d'onde λ (en nm) et d'émission (B) en fonction de la longueur d'onde λ (en nm) du composé suivant dans de l'acétonitrile : 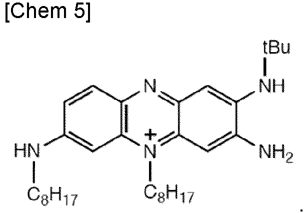

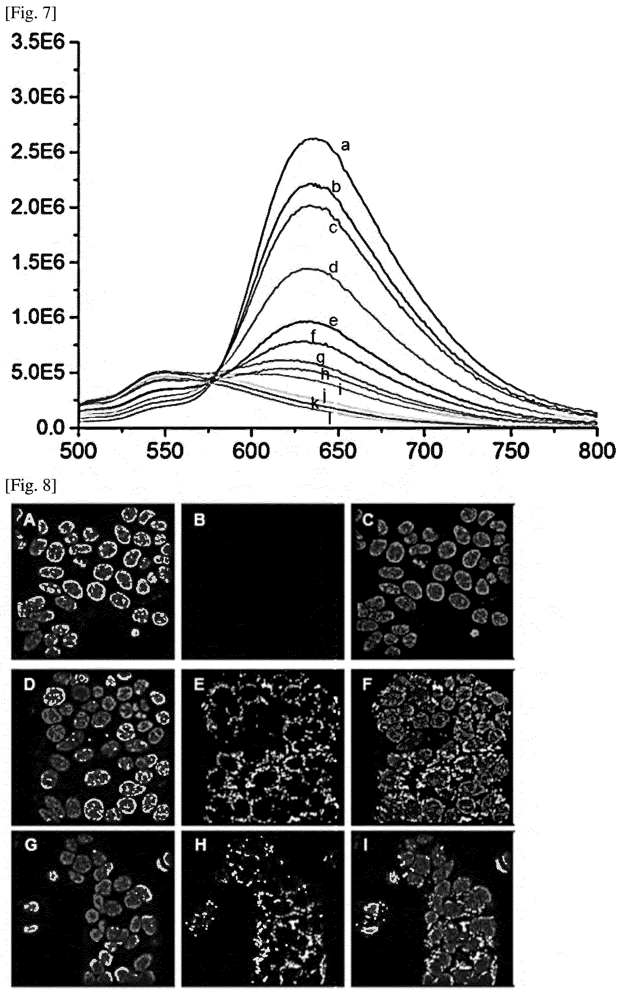

[Fig. 7] La figure 7 représente le spectre d'émission en fonction de la longueur d'onde λ (en nm) composé suivant dans de l'acétonitrile : 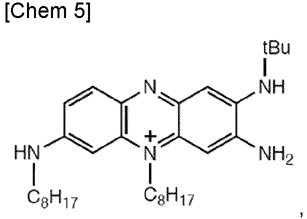 en présence de 0 équivalents de DBU (a), 0,1 équivalents de DBU (b), 0.2 équivalents de DBU (c), 0,4 équivalents de DBU (d), 0,6 équivalents de DBU (e), 0,8 équivalents de DBU (f), 1,0 équivalents de DBU (g), 1,2 équivalents de DBU (h), 1,4 équivalents de DBU (i), 1,6 équivalents de DBU (j), 1,8 équivalents de DBU (k), 2,5 équivalents de DBU (l).
[Fig. 8] La figure 8 représente la capture du composé de formule 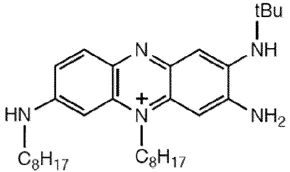 Par des cellules humaines de cancer sein (MCF-7). Les cellules MCF-7 ont été incubées pendant 16 h avec le composé aux concentrations de 0 µM (B), 0,1 µM (E) ou 0,5 µM (H). Les noyaux furent colorés au Hoechst 33342 (A, D et G). L'imagerie par fluorescence monophotonique a été réalisée sur des cellules vivantes à la longueur d'onde d'excitation de 514 nm avec un microscope Carl Zeiss. Les images C, F, et I représente la superposition des signaux des images A+B, D+E et G+H respectivement.

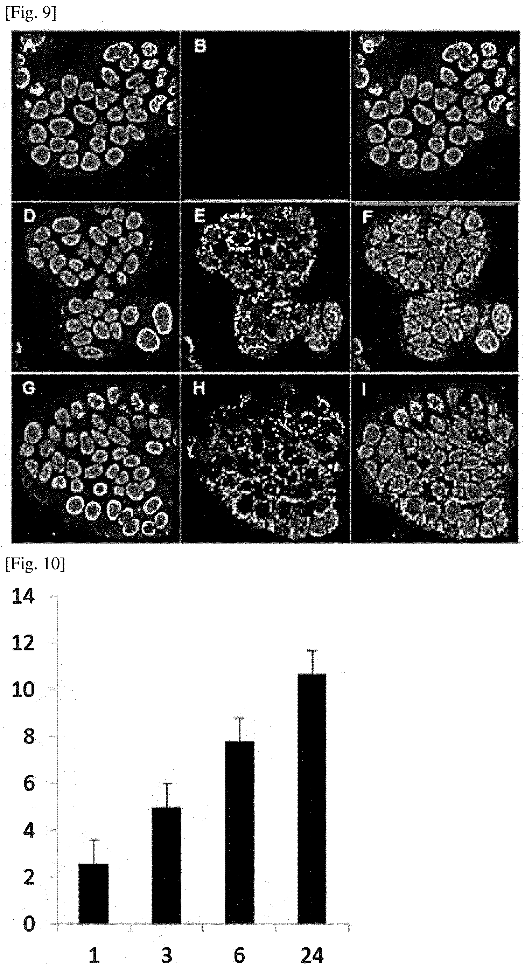

[Fig. 9] La figure 9 représente la capture du composé de formule 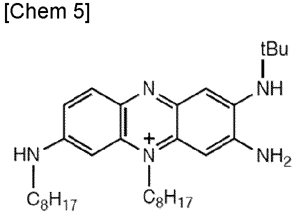 Par des cellules humaines de cancer sein (MCF-7). Les cellules MCF-7 ont été incubées pendant 16 h avec le composé aux concentrations de 0 µM (B), 0,5 µM à 790 nm (E) ou 0,5 µM à 810 nm (H). Les noyaux furent colorés au Hoechst 33342 (A, D et G). L'imagerie par fluorescence monophotonique a été réalisée sur des cellules vivantes aux longueurs d'onde d'excitation de 790 ou 810 nm avec un microscope Carl Zeiss. Les images C, F, et I représente la superposition des signaux des images A+B, D+E et G+H respectivement.
[Fig. 10] La figure 10 représente la cinétique d'incorporation du composé décrit à la figure 8 en utilisant un lecteur de plaque CLARIOstar afin de quantifier l'internalisation. Les données correspondent au pourcentage d'internalisation (fluorescence restante / fluorescence totale) en fonction du temps en heures. Les données correspondent aux moyennes de trois expériences ± l'écart type.

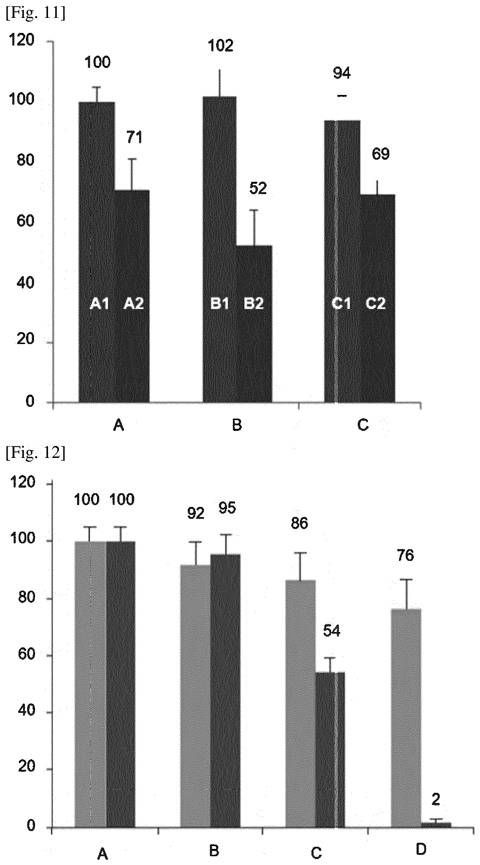

[Fig. 11] La figure 11 représente la survie des cellules MCF7 incubées pendant 5h avec le composé décrit à la figure 9 à une concentration de à 0,5µM, sans irradiation (A2), après irradiation à 790 nm (B2) ou 810 nm (C2). En contrôle, des cellules MCF7 non traitées sans irradiation (A1), après irradiation à 790 nm (B1) ou 810 nm (C1) sont présentées. L'axe de Y représente le pourcentage de cellules MCF-7 vivantes (les valeurs chiffrées sont indiquées au-dessus de chaque barre). Les données correspondent aux moyennes de trois expériences ± l'écart type.
[Fig. 12] La figure 12 représente un histogramme montrant l'efficacité de la thérapie photodynamique monophotonique à 540 nm. Les cellules MCF-7 ont été incubées avec le composé pendant 5 h à des doses de 0 nM (A), 1 nM (B), 10 nM (C) ou 100 nM (D) et irradiées (colonnes noires) ou non (colonnes grises) à 530 nm pendant 20 minutes. Deux jours plus tard, les cellules furent soumises à un test colorimétrique de viabilité cellulaire (MTT). L'expérience a été réalisée 3 fois. L'axe des Y représente le pourcentage des cellules MCF-7 vivantes.

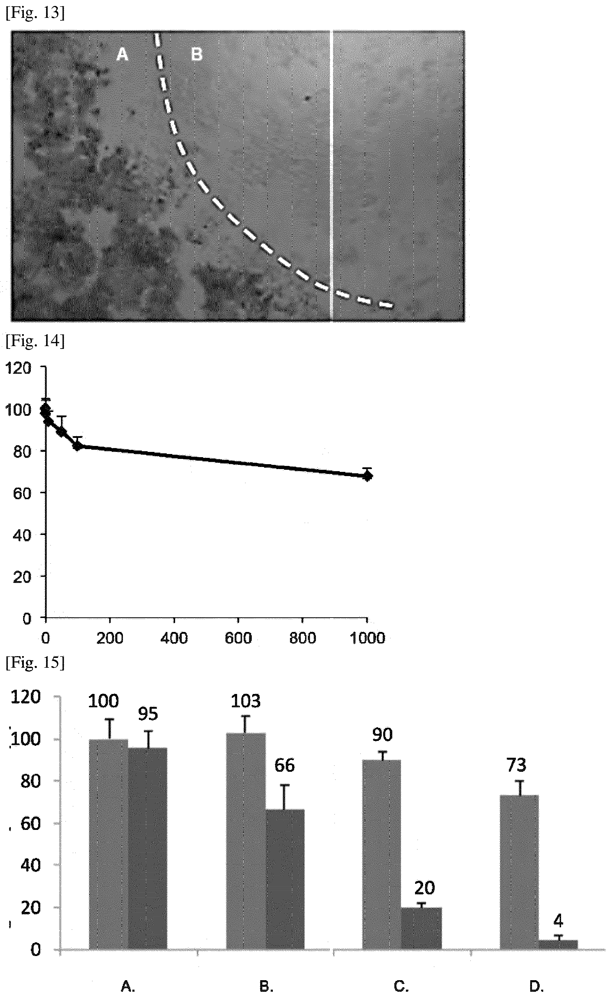

[Fig. 13] La figure 13 représente une photo de la zone de jonction entre des cellules MCF-7 traitées avec le composé à 100 nM et irradiées à 540 nm (B) ou non (A). Les cellules vivantes sont visibles grâce au marquage violet suite au traitement MTT.
[Fig. 14] La figure 14 représente un graphique montrant le pourcentage de cellules MCF-7 après traitement pendant 72h avec un composé selon l'invention à des doses indiquées. Les données correspondent aux moyennes de trois expériences ± l'écart type.
[Fig. 15] La figure 15 représente un histogramme montrant l'efficacité de la thérapie photodynamique monophotonique à 540 nm. Des cellules de kératose ont été incubées avec le composé de formule 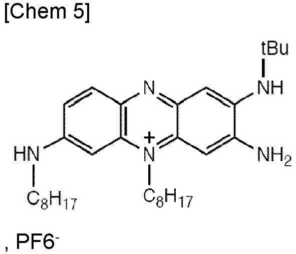 pendant 20 min à des doses de 0 nM (A), 10 nM (B), 25 nM (C) ou 50 nM (D) et irradiées (colonnes noires) ou non (colonnes grises) à 530 nm. Deux jours plus tard, les cellules furent soumises à un test colorimétrique de viabilité cellulaire (MTT). L'expérience a été réalisée 3 fois. L'axe des Y représente le pourcentage des cellules de kératose vivantes.

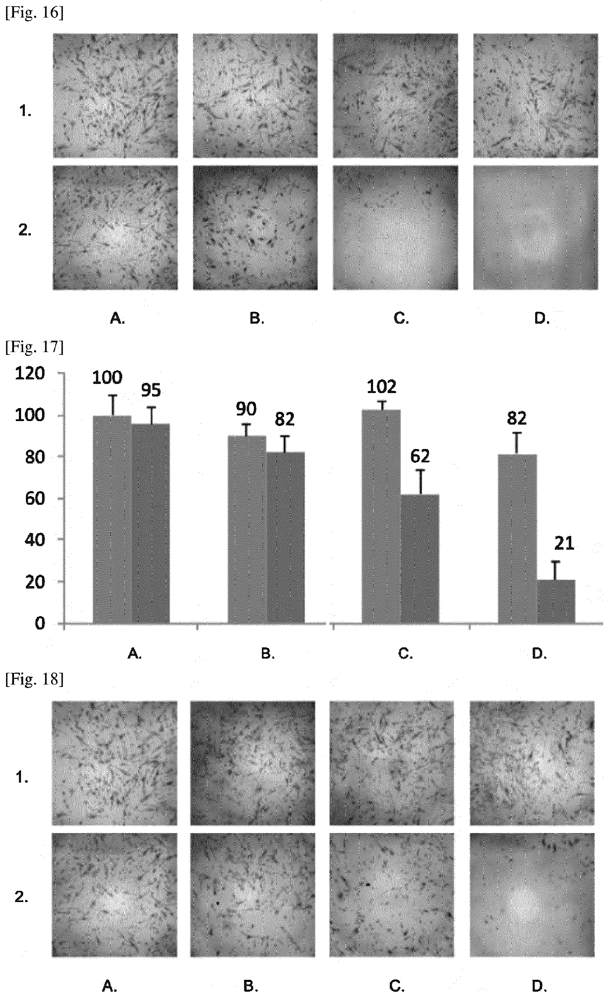

[Fig. 16] La figure 16 représente les photos de la zone non traitée au laser (1.) ou traitée au laser (2.) à des doses de 0 nM (A), 10 nM (B), 25 nM (C) ou 50 nM (D) de composé de formule . 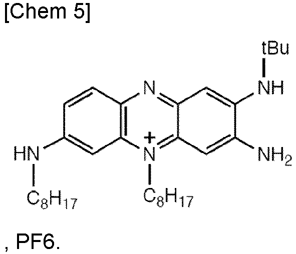
[Fig. 17] La figure 17 représente un histogramme montrant l'efficacité de la thérapie photodynamique monophotonique à 540 nm. Des cellules de kératose ont été incubées avec le composé de formule : 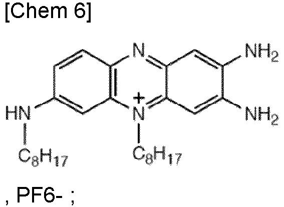 pendant 20 minutes h à des doses de 0 nM (A), 10 nM (B), 25 nM (C) ou 50 nM (D) et irradiées (colonnes noires) ou non (colonnes grises) à 530 nm. Deux jours plus tard, les cellules furent soumises à un test colorimétrique de viabilité cellulaire (MTT). L'expérience a été réalisée 3 fois. L'axe des Y représente le pourcentage des cellules de kératose vivantes.
[Fig. 18] La figure 18 représente les photos de la zone non traitée au laser (1.) ou traitée au laser (2.) à des doses de 0 nM (A), 10 nM (B), 25 nM (C) ou 50 nM (D) de composé de formule : 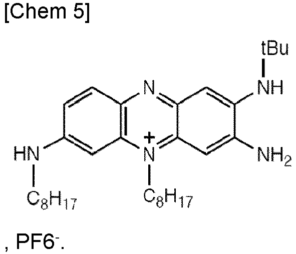

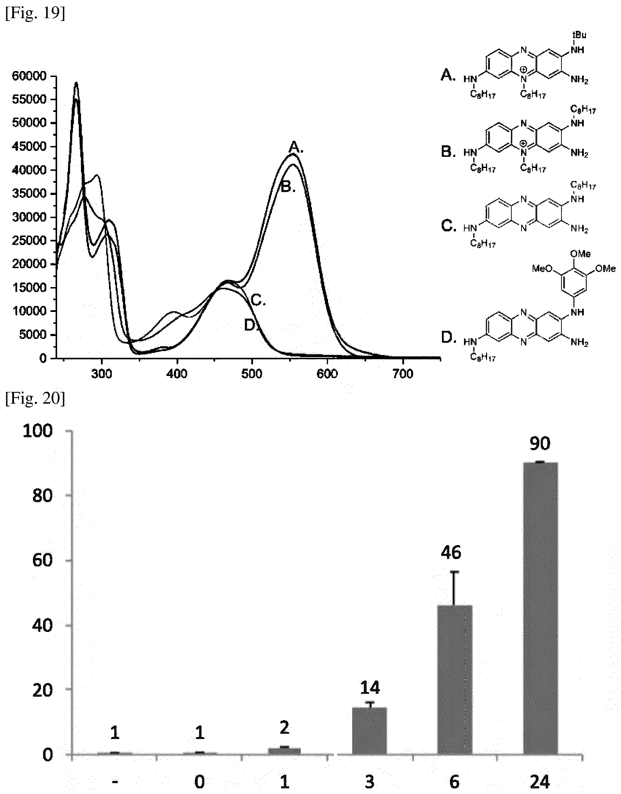

[Fig. 19] La figure 19 représente les spectres d'absorption des composés phénaziniums selon l'invention (A et B) et des composés phénazines de l'art antérieur (C et D).
[Fig. 20] La figure 20 représente un graphique montrant l'internalisation du composé de formule 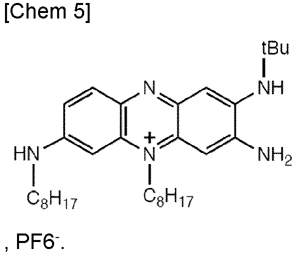 par des cellules vivantes cancéreuses MCF-7. Les cellules ont été traitées avec 0,5 nM de composé pendant 1, 3, 6 ou 24 h et la fluorescence rouge a été mesurée par cytométrie de flux. Les résultats montrent la moyenne de deux expériences +/- la déviation standard. L'axe des abscisses représente le temps d'incubation en heures et l'axe des ordonnées le pourcentage de cellules fluorescentes rouges.

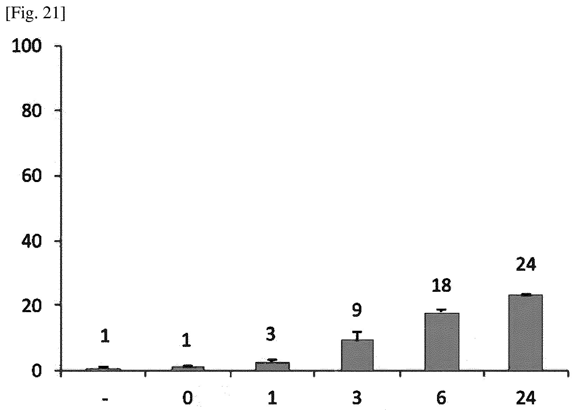

[Fig. 21] La figure 21 représente un graphique montrant l'internalisation du composé de formule 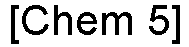 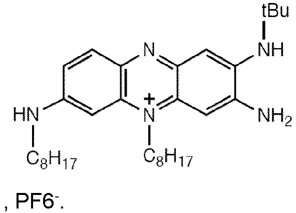 par des cellules vivantes saines de type fibroblastes. Les cellules ont été traitées avec 0,5 nM de composé pendant 1, 3, 6 ou 24 h et la fluorescence rouge a été mesurée par cytométrie de flux. Les résultats montrent la moyenne de deux expériences +/- la déviation standard. L'axe des abscisses représente le temps d'incubation en heures et l'axe des ordonnées le pourcentage de cellules fluorescentes rouges.

### EXEMPLES

Les réactifs commerciaux de qualité analytique ont été obtenus à partir de fournisseurs et utilisés directement sans purification supplémentaire. Les spectres RMN ¹H et ¹³C sont enregistrés dans du du CDCl₃, du CD₂Cl₂, du CD₃CN, de l'acétone-d₆ et du DMSO-d₆, déterminés avec un spectromètre Brucker AC250 fonctionnant à 250 MHz ou avec un spectromètre Jeol ECS400 fonctionnant à 400 MHz. Les décalages chimiques sont exprimés en ppm et les constantes de couplage (J) sont en hertz. Les motifs de séparation sont conçus sous la forme de s, singulet; br s, singulet large; d, doublet; dd, doublet doublet; t, triplet; td, doublet triplet; qt, quintet.

Les analyses élémentaires et MS (spectrométrie de masse) ont été réalisées par la Spectropole De Marseille. Des analyses spectrales de masse ESI sont enregistrées avec un spectromètre de masse 3200 QIRAP (Applied Biosystems SCIEX). Des analyses spectrales de masse haut-résolution sont enregistrées avec un spectromètre de masse SYNAPT G2 HDMS (Waters)

Les chromatographies de colonnes flash préparatives ont été réalisées à l'aide d'un gel de silice (Merck) G60 230-240 mesh.

### Exemple 1 - synthèses de composés selon l'invention et composés de référence

### 1- Procédé de synthèse n°1 - composé de formule III (1a)

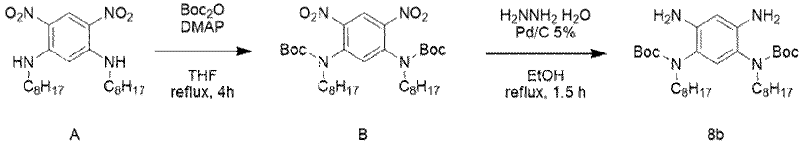

**Composé** A: A une solution de 1,5-difluoro-2,4-dinitrobenzène (DFNB) (m = 500 mg, 1,00 équiv.) dans de l'éthanol (v = 50 mL), ont été ajoutés du 1-Octylamine (v = 830 µL, 2.05 équiv.) et de la N, N-diisopropyléthylamine (DIPEA) (v = 874 µL, 2,05 équiv.). La solution a été chauffée jusqu'à reflux pendant 1,5 h. Après refroidissement à température ambiante, le solide résultant en suspension a été isolé par filtration, rincé avec de l'EtOH et séché sous vide pour obtenir le compose A (m = 1.04 g, rendement quantitatif) sous une forme cristalline orange.

¹H **NMR (250 MHz, CDCl₃):** δ 9.24 (s, 1H), 8.32 (br s, 2H), 5.65 (s, 1H), 3.27 (td, *Jₜ* = 7.0 Hz, *J_{d} =* 5.3 Hz, 4H), 1.82-1.71 (m, 4H), 1.54-1.29 (m, 20H), 0.89 (t, *J =* 6.8 Hz, 6H). ¹³C NMR (63 MHz, **CDCl₃):** δ 148.5, 129.5, 124.0, 90.0, 43.3, 31.7, 29.2, 29.1, 28.4, 27.1, 22.6, 14.0. ESI-MS: m/z [M+H]⁺ 423.3 (100%), [M+Na]⁺ 445.3 (6%), [M+K]⁺ 461.3 (4%); [M-H]⁻ 421.3 (100%). Analyse élémentaire pour C₂₂H₃₈N₄O₄: calculé. C 62.53, H 9.06, N 13.26, O 15.15; trouvé C 62.58, H 9.19, N 13. 21
**Composé B:** A une solution de composé A (m = 1,03 g, 2,43 mmol, 1,0 équiv.) dans du THF (10 mL), du Boc₂O (m = 1,94 g, 8,89 mmol, 3,7 équiv.) et du 4-diméthylaminopyridine (DMAP) (m = 50 mg, 0,41 mmol, 17%mol) ont été ajoutés. La solution a été chauffée à reflux pendant 4 h. Le solvant a été éliminé sous vide. Le produit brut a été purifié par chromatographie flash (silice F60, DCM 100) pour obtenir le composé B (m = 1.52 g, 2.44 mmol, rendement quantitatif) sous la forme d'un solide jaune.

**¹H NMR (250 MHz, CDCl₃):** δ 8.54 (s, 1H), 7.24 (s, 1H), 3.69 (m, 4H), 1.68-1.25 (m, 42H), 0.87 (t, *J =* 6.5 Hz, 6H). **¹³C NMR (63 MHz, CDCl₃):** 152.1, 142.5, 141.0, 127.8, 122.8, 82.9, 50.9, 31.7, 29.2, 29.2, 28.7, 27.9, 26.9, 22.6, 14.0. **MS: ESI-MS:** m/z [M+NH₄]⁺ 640.4 (100%), [M+Na]⁺ 645.4 (12%), [M+K]⁺ 661.3 (5%). **Analyse élémentaire** for C₃₂H₅₄N₄O₈: calculé. C 61.71, H 8.74, N 9.00, O 20.55; trouvé C 61.68, H 8.96, N 8.82.

**Composé 8b:** A une suspension de Pd sur du carbone carbon 5% (m = 180 mg, 0,085mmol, 1%mol) dans de l'EtOH (80 mL), du compose B (m = 5,15 g, 8,28 mmol, 1,0 équiv.) et de l'hydrazine monohydrate (2,3 mL, 47,1 mmol, 5,7 équiv.) ont été ajoutés. Le mélange a été chauffé à reflux pendant 1,5 h. Le Pd/C a été éliminé par filtration au travers de Celite 545, et la phase solide a été rincée avec du dichlorométhane (3 x100 mL). La phase organique combinée a été lavée avec de l'eau (3 x 150 mL) et de la saumure (100 mL), séchée avec du MgSO₄anhydre, filtrée, concentrée et séchée sous vide pour obtenir le composé **8b** (m = 4,43 g, 7,87 mmol, 96% de rendement) sous la forme d'un solide jaune.

**¹H NMR (250 MHz, CDCl₃):** δ 6.55 (br s, 1H), 6.09 (s, 1H), 3.58 (br s, 6H), 3.27 (m, 2H), 1.51-1.24 (m, 42H), 0.86 (t, *J =* 6.5 Hz, 6H). **¹³C NMR (63 MHz, CDCl₃):** 155.2, 142.7, 142.5, 129.5, 129.1, 118.9, 102.0, 79.2, 57.6, 48.4, 31.5, 29.1, 29.0, 28.0, 26.6, 22.3, 18.1, 13.8. **HRMS (ESI-TOF):** m/z [M+H]⁺ for C₃₂H₅₉N₄O₄⁺ calculé. 563.4531, trouvé 563.4532, err. < 1 ppm; m/z [M+NH₄]⁺ for C₃₂H₆₂N₅O₄⁺ calculé. 580.4796, trouvé 580.4803, err. < 2 ppm.

**Composé 12b** : A une solution de compose **8b** (m = 303 mg, 0,538 mmol) dans du dichlorométhane (v = 5 mL), HCl (12N, v = 2 mL) a été ajouté du TFA à 0°C. Ce mélange a été agité sous argon toute la nuit. Le solide résultant en suspension a été collecté par filtration, rincé avec du CH₂Cl₂ (v = 20 mL), et séché sous vide pour obtenir le composé **12b** (m = 196 mg, 0,449 mmol, 84% de rendement) sous la forme d'un solide rose clair. Ce produit brut a été utilisé directement sans plus de purification.

**¹H NMR (250 MHz, DMSO-d₆):** δ 6.85 (br s, 1H), 6.44 (br s, 1H), 3.06 (t, *³J_{HH} =* 7.2 Hz, 4H), 1.62 (m, 4H), 1.35-1.26 (m, 20H), 0.86 (t, *³J_{HH}* = 6.8 Hz, 6H). No ¹³C NMR spectrum could be recorded owing to the poor stability in solution. MALDI-TOF MS: m/z M^{+•} for C₂₂H₄₂N₄^{•+} calculé. 362.3, trouvé 362.3 (100%).

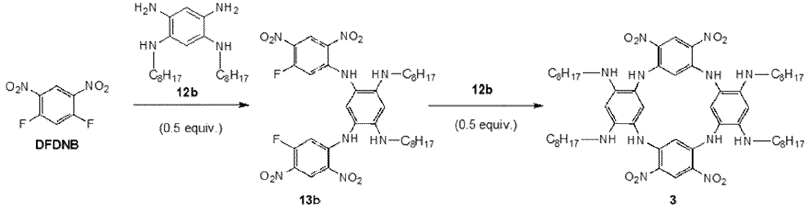

**Composé 13b** : A une solution de DFDNB (m = 258 mg, 1.27 mmol, 1.8 équiv.) dans du MeCN (v = 25 mL) a été ajouté du composé 12b (m = 306 mg, 0,703 mmol, 1,0 équiv.). Le ballon a été fermé avec un septum et la solution a été refroidie dans un bain d'eau glacée et dégazée. Du N(*i*Pr)₂Et (v = 735 µL, 4,22 mmol, 6,0 équiv.) a alors été ajouté goutte à goutte à l'aide d'une seringue sous argon. La solution a été agitée à 0 °C pendant 2 heures puis à température ambiante pendant deux heures additionnelles. La solution a été concentrée sous vide, et le résidu a été repris avec de l'EtOH (v = 30 mL) et du MeCN (v = 10 mL). Le solide obtenu en suspension a été récupéré par filtration, rincé avec de l'EtOH (v = 100 mL) et de l'Et₂O (v = 20 mL), et séché sous vide pour obtenir le composé 13b sous la forme d'une poudre orange (m = 365 mg, 0.499 mmol, 79% de rendement).

**¹H NMR (250 MHz, CDCl₃):** δ 9.29 (br s, 2H), 9.15 (d, *⁴J_{HF} =* 7.8 Hz, 2H), 6.84 (s, 1H), 6.52 (d, *³J_{HF} =* 13 Hz, 2H), 6.07 (s, 1H), 3.97 (br s, 2H), 3.19 (t, *³J_{HH}* = 7.0 Hz, 4H), 1.66-1.26 (m, 24H), 0.88 (t, *³J_{HH} =* 7.0 Hz, 6H). **¹H NMR (250 MHz, Acetone-d₆):** δ 9.60 (br s, 2H), 9.01 (d, *⁴J_{HF} =* 8.0 Hz, 2H), 7.08 (s, 1H), 6.70 *(d, ³J_{HH} =* 14.3 Hz, 2H), 6.20 (s, 1H), 5.13 (br s, 2H), 3.24 (t, *³J_{HH}* = 7.0 Hz, 4H), 1.66-1.55 (m, 4H), 1.29-1.26 (m, 20H), 0.87 (t, *³J_{HH} =* 7.0 Hz, 6H). **¹³C NMR (63 MHz, CDCl₃):** δ 159.9 (d, *¹J_{CF} =* 267 Hz), 150.0, 149.8, 146.1, 127.9, 127.6, 127.3 *(d, ²J_{CF} =* 10.1 Hz), 109.8, 103.7 (d, *²J_{CF}* = 27.7 Hz), 93.4, 43.5, 31.7, 29.28, 29.23, 29.18, 27.1, 22.6, 14.0. **HRMS (ESI-**TOF): m/z [M+H]⁺ for C₃₄H₄₅N₈O₈F₂⁺ calculé. 731.3323, trouvé 731.3323, err. < 1 ppm.

**Composé 3 :** A une solution de composé **13b** (m = 160 mg, 0,219 mmol, 1,0 équiv.) dans du MeCN anhydre (v = 30 mL), du composé **12b** (m = 115 mg, 0.263 mmol, 1.2 équiv.) a été ajouté. Le ballon a été fermé, dégazé et du N(*i*Pr)₂Et a été ajouté goutte à goutte (m = 370 µL, 2,12 mmol, 9,6 équiv.) à l'aide d'une seringue sous argon. Le mélange a été agité à température ambiante pendant 2 h sous agitation, puis chauffé à reflux toute la nuit. Après concentration du solvant sous vide, le résidu a été repris avec un mélange d'acétone (v = 5 mL) et d'éthanol (v = 5 mL). Le produit solide résultant a été isolé par filtration, lavé avec de l'EtOH, et séché sous vide pour obtenir le composé 3 (m = 155,5 mg, 0,148 mmol, 68% de rendement) sous la forme d'une poudre orange.

**¹H NMR (250 MHz, CDCl₃):** δ 9.26 (s, 2H), 8.85 (br s, 4H), 6.58 (s, 2H), 5.82 (s, 2H), 5.49 (s, 2H), 3.91 (br t, *³J_{HH} =* 5.4 Hz, 4H), 3.09-2.99 (m, 8H), 1.53-1.28 (m, 48H), 0.89 (t, *³J_{HH}* = 6.8 Hz, 12H). **¹³C NMR (63 MHz, CDCl₃):** δ 149.9, 146.4, 129.2, 129.0, 125.4, 110.6, 95.7, 92.6, 43.8, 31.8, 29.49, 29.46, 29.33, 27.3, 22.7, 14.1. **HRMS (ESI-TOF):** m/z [M+H]⁺ for C₅₆H₈₅N₁₂O₈⁺ calculé. 1053.6608, trouvé 1053.6608, err. < 1 ppm.

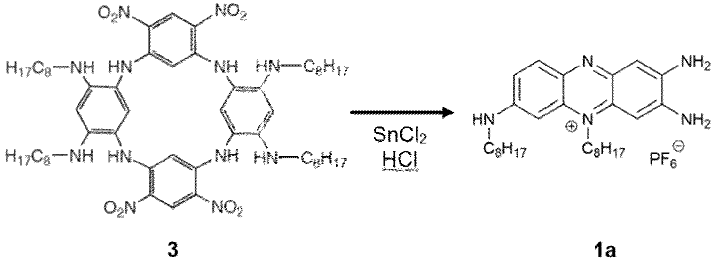

**Composé 1a** : A une solution de macrocycle 3 (50 mg, 0,05 mmol, 1 équiv.) dans de l'éthanol absolu (50 mL), a été ajouté du SnCl₂·2H₂O (343 mg, 1,52 mmol, 32 équiv.) et du HCl (12M, 0,13 mL). Le mélange a été agité à reflux toute la nuit et neutralisé avec du NaHCO₃ avant d'ajouter de l'éthanol (30 mL) et de l'eau (20 mL). Après évaporation du solvant sous pression réduite, le résidu a été extrait avec un mélange dichlorométhane/éthanol (3/1, v/v). La couche organique rouge a été lavée avec une solution aqueuse de HPF₆ (1 poids.% dans de l'eau, 4 x 150 mL) et de la saumure (100 mL), séché avec du MgSO₄ et concentré sous vide pour obtenir le composé **1a [PF₆]** en tant que solide rouge foncé (35 mg, 62% rendement).

**¹H NMR (400 MHz, CD₃CN)**: δ 7.82 (d, *J =* 9.3 Hz, 1H), 7.23 (dd, *J =* 9.3 Hz, *J =* 2.2 Hz, 1H), 7.18 (s, 1H), 6.99 (s, 1H), 6.55 (d, *J =* 2.2 Hz, 1H), 6.33 (br t, *J =* 5.2 Hz, 1H), 6.12 (br s, 2H), 4.84 (br s, 2H), 4.57 (t, *J* = 8.2 Hz, 2H), 3.37 (td, *J =* 6.9 Hz, *J =* 6.0 Hz, 2H), 1.72 (quint, *J* = 7.3 Hz, 2H), 1.61 (quint, *J* = 7.8 Hz, 2H), 1.47 - 1.27 (m, 20H), 0.91 - 0.87 (m, 6H). **HRMS (ESI-TOF):** m/z [M+NH₄]⁺ for C₂₈H₄₄N₅⁺ calculé. 450.3591, trouvé 450.3592, err. < 1 ppm.

### 2- Procédé de synthèse n°2 - composé de formule IV (1c)

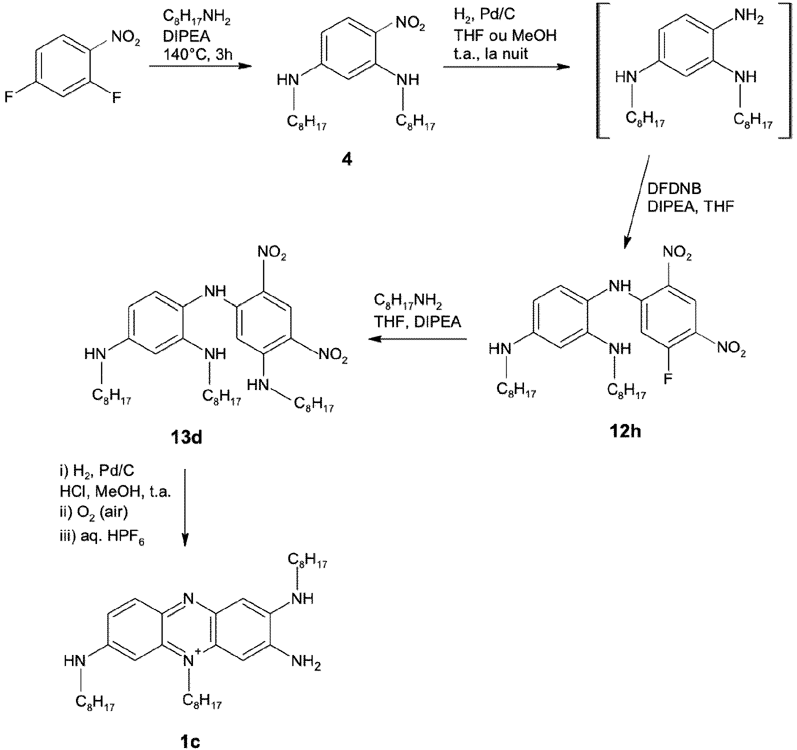

**Composé 4** : Du 2,4-difluoronitrobenzène (6,5 mL, 0,059 mol, 1 équiv.), du 1-octylamine (40 mL, 0.243 mol, 4.1 équiv.) et du DIPEA (18 mL, 0,101 mol, 1,7 équiv.) ont été introduits dans une bombe a pression qui a été fermée avec un bouchon de Teflon seal. Le mélange a été chauffé jusqu'à 145 °C pendant 3 h. Après refroidissement à température ambiante, 15 mL d'éthanol ont été ajoutés. Cette suspension a été triturée par ultrasons. Le produit solide résultant en suspension a été isolé par filtration, rincé à l'eau chaude, et séché sous vide pour obtenir le composé **4** sous la forme d'une poudre jaune (21.6 mg, 96% de rendement).

**¹H NMR (250 MHz, CDCl₃):** δ 8.52 (br s, 1H), 7.99 (d, *J =* 9.3 Hz, 1H), 5.89 (dd, *J =* 9.3 Hz, *J =* 2.3 Hz, 1H), 5.62 (d, *J* = 2.3 Hz, 1H), 4.52 (br s, 1H), 3.23 - 3.14 (m, 4H), 1.75 - 1.62 (m, 4H), 1.42 - 1.28 (m, 20H), 0.91 - 0.85 (m, 6H). **¹³C NMR (63 MHz, CDCl₃):** 154.4, 148.6, 129.2, 123.6, 104.7, 89.8, 43.3, 42.9, 31.8, 31.7, 29.3, 29.2, 29.1, 28.8, 27.1, 27.0, 22.6, 14.0. MS: ESI-MS: m/z [M+H]⁺ 378.3 (100%); [M-H]⁻376.3 (100%), [M+CH₃COO]⁻ 436.3 (48%). **Analyse élémentaire pour** C₂₂H₃₉N₃O₂·¹/₅·C₂H₅OH: calculé. C 69.56, H 10.48, N 10.86, O 9.10; trouvé C 69.41, H 10.39, N 10.92.

**Composé 12h** : une solution de composé 4 (628 mg, 1,66 mmol, 1 équiv.) dans du THF (25 mL) a été hydrogénée (40 bars) toute la nuit en présence de Pd/C (5 poids.%, 36 mg, 0,02 mmol, 1 mol%). Après réduction de la pression, la solution a été dégazée sous sonication pendant 5 min. Du 1,5-difluoro-2,4-dinitrobenzene (320 mg, 1,58 mmol, 0.95 équiv.) a été ajouté à la solution sous agitation à 0 °C. La solution a été laisse à cette température pendant 10 min supplémentaires et l'achèvement de la réaction a été suivi par TLC. Alors du DIPEA (301 µL, 1,66 mmol, 1 équiv.) a été ajouté pour neutraliser la solution. Le Pd/C a été éliminé par filtration au travers de celite. Le produit brut a été purifié par chromatographie flash sur silicagel en utilisant un mélange dichlorométhane/cyclohexane (1/1) comme éluant pour obtenir le composé 12h sous la forme d'un solide rouge (620 mg, 75% de rendement).

**¹H NMR (400 MHz, CDCl₃):** δ 9.31 (s, 1H), 9.15 (d, *J* = 7.7 Hz, 1H), 6.83 (d, *J =* 8.4 Hz, 1H), 6.53 (d, *J =* 13.4 Hz, 1H), 6.00 (dd, *J* = 8.4, 2.3 Hz, 1H), 5.95 (d, *J =* 2.1 Hz, 1H), 3.77 (s, 1H), 3.67 (s, 1H), 3.11 (td, *J =* 14.1, 8.4 Hz, 4H), 1.65 (quintet, *J* = 7.2 Hz, 2H), 1.56 (quintet, *J =* 7.1 Hz, 2H), 1.36 (m, 20H), 0.89 (t, *J =* 6.7 Hz, 3H), 0.87 (t, *J* = 6.7 Hz, 3H). ¹³C **NMR (100 MHz, CDCl₃):** δ 159.9 (d, *J_{C-F}* = 270.7 Hz), 150.5, 150.3, 150.2, 145.1, 128.5, 127.7, 127.7, 127.6, 126.9 (d, *J_{C-F} =* 10.2 Hz), 110.7, 103.9 (d, *J_{C-F}* = 27.4 Hz), 101.6, 95.1, 43.9, 43.4, 31.8, 31.8, 31.8, 29.5, 29.4, 29.3, 29.3, 29.3, 29.3, 29.2, 29.2, 27.2, 27.1, 22.7, 22.6, 14.1, 14.1. **HRMS (ESI-TOF):** m/z [M+H]⁺ pour C₂₈H₄₃FN₅O₄⁺ calculé. 532.3294, trouvé 532.3281, err. < 2 ppm.

**Composé 13d** : une solution de composé 4 (628 mg, 1,66 mmol, 1 équiv.) dans du THF (25 mL) a été hydrogénée (40 bars) toute la nuit en présence de Pd/C (5 poids.%, 36 mg, 1 mol%). Après diminution de la pression, la solution a été dégazée par sonication pendant 5 min, puis refroidie à 0°C dans un bac à glace, et du 1,5-difluoro-2,4-dinitrobenzene (320 mg, 1,58 mmol, 0.95 équiv.) a été ajouté dans la solution sous agitation. La réaction a été maintenue à 0 °C pendant 10 min. Alors du 1-octylamine (286 µL, 1,93 mmol, 1.1 équiv.) et du DIPEA (289 µL, 1,66 mmol, 1 équiv.) ont été ajoutés. Le mélange a été agité à température ambiante pendant 3 jours. Après filtration au travers de celite et concentration, le produit brut a été purifié par flash chromatographie sur silicagel en utilisant un mélange dichlorométhane/cyclohexane (50/50 à 55/45) en tant qu'éluant pour obtenir le composé **13d** sous la forme d'un solide rouge (498 mg, 49% de rendement).

**TLC:** R_{f} = 0.27 (SiO₂ F60, dichloromethane/cyclohexane, 7/3). **¹H NMR (400 MHz, CDCl₃):** δ 9.27 (s, 1H), 9.12 (br s, 1H), 8.21 (br t, *J =* 4.8 Hz, 1H), 6.87 (d, *J =* 8.3 Hz, 1H), 6.00 (dd, J = 8.3, 2.2 Hz, 1H), 5.96 (d, *J =* 2.2 Hz, 1H), 5.69 (s, 1H), 3.77 (br s, 1H), 3.68 (br s, 1H), 3.13 (t, *J =* 7.2 Hz, 2H), 3.13 - 3.05 (m, 2H), 3.00 (q, *J =* 6.1 Hz, 2H), 1.68 - 1.52 (m, 6H), 1.45 - 1.23 (m, 30H), 0.90 - 0.84 (m, 9H). **¹³C NMR (100 MHz, CDCl₃):** δ 149.5, 149.0, 148.4, 145.2, 129.4, 128.6, 124.8, 124.3, 112.0, 101.5, 95.2, 93.0, 44.1, 43.5, 43.1, 31.8, 31.8, 31.7, 29.6, 29.4, 29.4, 29.3, 29.2, 29.2, 29.2, 29.1, 28.2, 27.2, 27.1, 26.9, 22.6, 22.6, 22.6, 14.1, 14.1, 14.0. **HRMS (ESI-TOF):** m/z [M+I]⁻ for C₃₆H₆₀N₆O₄I⁻ calculé. 767.3726, trouvé 767.3725, err. < 2 ppm.

**Composé 1c :** Une solution de composé 13d (200 mg, 0,31 mmol, 1 équiv.) dans du méthanol (40 mL) a été hydrogénée (40 bars) toute la nuit en présence de Pd/C (5 poids.%) et d'HCl (12M, 0.1 mL). Alors le mélange a été agité à l'air pendant 24h. Le Pd/C a été retiré par filtration au travers de celite. Après retrait du solvent sous pression réduite, le solide résultant a été repris avec du dichlorométhane (80 mL), lavé avec une solution de HPF₆ aqueux (1 poids.% dans de l'eau, 2 x 50 mL) puis de l'eau distillées (50 mL) et in fine concentré. Le résidu a été purifié par flash chromatographie sur alumina 90 standard en utilisant un mélange dichlorométhane/cyclohexane (100/0 à 99/1) en tant qu'éluant pour obtenir le composé **1c[PF₆⁻]** en tant que solide rouge (192 mg, 87% de rendement).

**¹H NMR (400 MHz, CD₃CN):** δ 7.78 (d, *J =* 9.2 Hz, 1H), 7.19 (dd, J = 9.2Hz, 1.7Hz, 1H), 6.96 (s, 1H), 6.89 (s, 1H), 6.52 (d, *J =* 1.7Hz, 1H), 6.24 (br t, *J =* 5.2 Hz, 1H), 6.18 (br s, 2H), 4.78 (br s, 1H), 4.54 (t, *J* = 8.1 Hz, 2H), 3.34 (td, J = 6.7 Hz, 6.0 Hz, 2H), 3.28 (td, *J =* 6.7 Hz, 5.2 Hz, 2H), 1.79 - 1.67 (m, 4H), 1.64 - 1.56 (m, 2H), 1.51 - 1.30 (m, 30H), 0.91 - 0.88 (m, 9H). **¹³C NMR (100 MHz, DMSO-d₆):** δ = 152.6, 150.3, 138.7, 138.3, 134.9, 132.2, 131.3, 130.2, 102.5, 92.3, 47.1, 43.1, 42.5, 31.2, 31.2, 28.8, 28.8, 28.7, 28.6, 28.0, 27.6, 26.7, 26.6, 26.2, 26.0, 22.0, 13.9, 13.9. **HRMS (ESI-TOF):** m/z [M+H]⁺ for C₃₆H₆₀N₅⁺ calculé. 562.4843, trouvé 562.4855, err. < 3 ppm.

### Synthèse de composés de référence ne faisant pas partie de l'invention où R2 est un hydrogène

### Composé de référence PR4

### Composé 12: 5-fluoro-2-nitroaniline

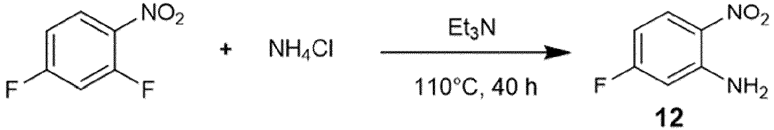

Du DFDNB (1,0 mL, 9,11 mmol, 1,0 eq), du NH₄Cl (975 mg, 18,2 mmol, 2,0 eq) et de la triéthylamine (9,0 mL) ont été introduits dans une bombe à pression. La bombe a été fermée avec un bouchon de Teflon. Le mélange a été laisse à chauffer jusqu'à 110°C pendant 40 hrs. Après refroidissement à température ambiante, le produit brut a été placé dans un mélange de DCM (150 mL) et d'eau (150 mL). Pendant l'agitation, 12N de HCl ont été ajoutés goutte à goutte jusqu'à ce que le pH de la phase aqueuse atteigne environ 1. La phase organique a été séparée et lavée avec de l'eau (150 mL), séchée par du MgSO₄. De l'heptane additionnel (40 mL) a été ajouté à cette solution. Le solvant a été éliminé en baissant la pression. Les résidus ont été séchés sous vide pour obtenir le composé **12** (1,25 g, 8,01 mmol, 88% de rendement) sous la forme d'une poudre jaune. ¹H NMR (400 MHz, **CDCl₃):** δ 8.184 (dd, *J =* 9.6 Hz, *J =* 5.6 Hz, 1H), 6.449-6.41 (m, 2H), 6.200 (br s, 2H) ¹³C NMR (100 MHz, **CDCl₃):** δ 168.2 (d, *J=* 260 Hz), 146.7, 146.6, 129.4 (d, *J =* 12 Hz), 105.9 (d, *J =* 25 Hz), 103.8 (d, *J=* 26 Hz). MS: ESI-MS: m/z [M+Li]⁺ 157.1 (26%), [M+Li]⁺ 163.1 (100%); [M-H]⁻ 154.9 (100%). Analyse élémentaire pour C₆H₅FN₂O₂: calculé. C 46.16, H 3.23, F 12.17, N 17.94, O 20.50; trouvé C 46.63, H 3.15, N 17.90.

### Composé 13: 4-Nitro-N¹-octylbenzene-1,3-diamine

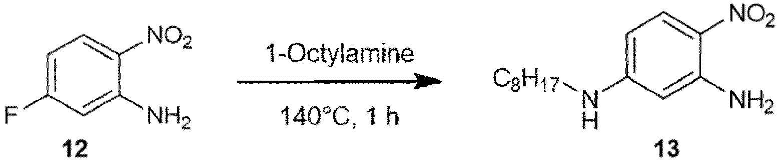

Du composé 12 (808 mg, 5,18 mmol, 1.0 eq) et du 1-octylamine (3,0 mL, 18,2 mmol, 3,5 eq) ont été introduits dans une bombe à pression. La bombe a été fermée avec un bouchon de Teflon. Le mélange a été agité à 140°C pendant 1 h. Après refroidissement à température ambiante, de l'heptane (10 mL) a été ajouté. Le solide résultant en suspension a été isolé par filtration, purifié par chromatographie (silice 60F, DCM, 100) pour obtenir le composé **13** (1,23 g, 4,64 mmol, 90% de rendement) sous la forme d'un solide jaune.

¹H NMR (250 MHz, **CDCl₃):** δ 7.96 (d, *J* = 9.3 Hz, 1H), 5.97 (dd, *J =* 9.3 Hz, *J =* 2.5 Hz, 1H), 5.71 (d, *J =* 2.5 Hz, 1H), 3.15 (t, *J =* 7.1 Hz, 2H), 1.67 (quint, *J =* 7.1 Hz, 2H), 1.40-1.28 (m, 10H), 0.90 (t, *J* = 7.1 Hz, 3H). ¹³C NMR (63 MHz, **CDCl₃):** δ 153.9, 147.9, 128.4, 124.2, 106.2, 94.8, 43.4, 31.7, 29.2, 29.1, 29.0, 27.0, 22.6, 14.0 MS: ESI-MS: m/z [M+H]⁺ 266.3 (100%), [M+Li]⁺ 272.3 (19%), [M+Na]⁺ 288.3 (4%); [M-H]⁻264.1 (100%). Analyse élémentaire pour C₁₄H₂₃N₃O₂: calculé. C 63.37, H 8.74, N 15.84, O 12.06; trouvé C 63.65, H 8.83, N 15.66.

### Composé 14: N⁴-octylbenzene-1,2,4-triamine

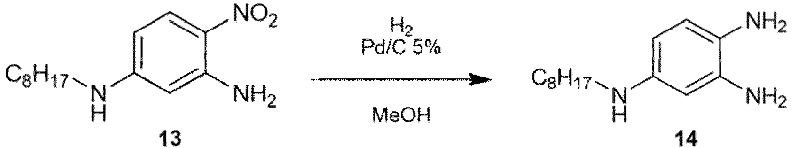

Une solution de composé 13 (975 mg, 3.67 mmol) dans du MeOH (75 mL) a été hydrogéné (40 bars) en présence de Pd/C (5%) toute la nuit. Le Pd/C a été alors retiré par filtration au travers de Celite. Après concentration sous pression réduite et séchage sous vide, le composé **14** a été obtenu sous la forme d'un solide vert profond (870 mg, 3,69 mmol, rendement quantitatif). ¹H NMR (400 MHz, **CDCl₃):** δ 6.60 (d, *J* = 8 Hz, 1H), 6.07 (d, *J* = 2.4 Hz, 1H), 6.03 (dd, *J* = 8 Hz, *J* = 2.4 Hz, 1H), 3.11 (br s, 5H), 3.03 (t, *J* = 7.2 Hz, 2H), 1.61 (quint, *J =* 7.2 Hz, 2H), 1.38-1.28 (m, 10H), 0.90 (t, *J =* 6.8 Hz, 3H)

¹³C NMR (100 MHz, **CDCl₃):** 143.5, 137.4, 124.8, 119.4, 104.7, 102.1, 45.1, 31.8, 29.6, 29.4, 29.3, 27.2, 22.6, 14.1. Analyse élémentaire pour C₁₄H₂₅N₃: calculé. C 71.44, H 10.71, N 17.85; trouvé C 71.23, H 10.82, N 17.88.

### Composé 20: N¹-(2,4-dinitro-5-(octylamino)phenyl)-N⁴-octylbenzene-1,2,4-triamine

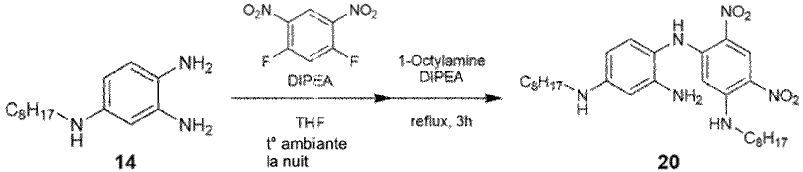

A une solution de composé **14** (860 mg, 3,65 mmol, 1,00 eq) dans du THF (40 mL), du DFDNB (671,5 mg, 3,29 mmol, 0,90 eq) a été ajouté. Le ballon a été scellé et dégazé par 3 cycles de pompe-argon. Du DIPEA dégazé (640 µL, 3,67 mmol, 1,00 eq) a été ajouté goutte à gouttes à l'aide d'une seringue sous argon. Le mélange a été agité à température ambiante toute la nuit. Du 1-octylamine (604 µL, 3,65 mmol, 1.00 eq) et du DIPEA additionnel (660 µL, 3,79 mmol, 1,04 eq) ont été ajoutés. La solution a été chauffée jusqu'au reflux pendant 3 h et refroidie à température ambiante. Après concentration du solvant sous vide, le produit solide résultant a été isolé par filtration avant l'ajout d'EtOH (40 mL), rincé avec de l'EtOH (6 x 10 mL) et séché sous vide pour obtenir le composé **20** (1,36 g, 2,57 mmol, 79% de rendement) sous la forme d'une poudre jaune. ¹H NMR (400 MHz, **CDCl₃):** δ 9.27 (s, 1H), 9.19 (br s, 1H), 8.23 (br t, *J* = 4.8 Hz, 1H), 6.89 (d, *J* = 8.4 Hz, 1H), 6.11 (dd, *J* = 8.4 Hz, *J* = 2 Hz, 1H), 6.06 (d, *J =* 2 Hz, 1H), 5.71 (s, 1H), 3.69 (br s, 1H), 3.65 (br s, 2H), 3.11 (t, *J =* 7.2 Hz, 2H), 3.06 (td, *J =* 6.8 Hz, *J* = 5.6 Hz, 2H), 1.67-1.57 (m, 4H), 1.42-1.27 (m, 20H), 0.90-0.87 (m, 6H). ¹³C NMR (100 MHz, **CDCl₃):** δ 149.3, 148.8, 148.5, 143.8, 129.5, 129.0, 124.9, 124.3, 112.3, 104.6, 99.0, 92.9, 44.0, 43.1, 31.8, 31.7, 29.5, 29.4, 29.3, 29.2, 29.1, 28.3, 27.2, 26.9, 22.6, 14.1. HRMS (ESI-TOF): m/z [M+H]⁺ for C₂₈H₄₅N₆O₄⁺ calculé. 529.3497, trouvé 529.3493, err. < 1 ppm.

### Composé PR4 de référence : N²,N⁷-dioctylphenazine-2,3,7-triamine

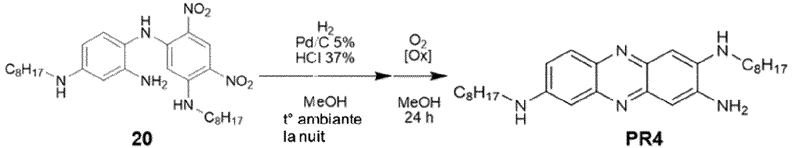

Une solution de composé **20** (302 mg, 0,571 mmol) dans du MeOH (30 mL) a été hydrogénée (20 bars) en présence de Pd/C (5%) et de HCl (12M, 0,5 mL) toute la nuit. Après ajout de MeOH (30 mL), la solution a été agitée sous air pendant 24 h. Le Pd/C a été retiré par filtration sous Celite, et la phase solide a été rincée avec du MeOH (400 mL). La solution a été neutralisée avec du NaHCO₃ jusqu'à pH 9 avant d'ajouter de l'eau (100 mL). Le solvant a été concentré jusqu'à environ 100 mL sous pression réduite. Après refroidissement à 5 °C toute la nuit, le solide résultant en suspension a été isolé par filtration et lavé avec de l'eau (2 x 30 mL), séché sous vide pour obtenir le composé **PR4** (238 mg, 0,529 mmol, 93% de rendement) sous la forme d'une poudre rouge. ¹H NMR (400 MHz, **DMSO-d₆):** δ 7.59 (d, *J* = 9.2 Hz, 1H), 7.11 (dd, *J* = 9.2 Hz, *J =* 2.4 Hz, 1H), 6.79 (s, 1H), 6.59 (s, 1H), 6.58 (d, *J* = 2.4 Hz, 1H), 6.22 (br t, *J* = 4.8 Hz, 1H), 5.98 (br s, 2H), 5.65 (br t, *J* = 4.4 Hz, 2H), 3.22 (td, *J =* 6.8 Hz, *J =* 5.2 Hz, 2H), 3.13 (td, *J =* 6.8 Hz, *J =* 5.6 Hz, 2H), 1.73-1.60 (m, 4H), 1.43-1.27 (m, 20H), 0.57-0.56 (m, 6H). ¹³C NMR (100 MHz, **DMSO-d₆):** 147.6, 143.0, 141.2, 140.2, 138.5, 135.5, 128.2, 121.1, 102.5, 99.9, 99.4, 43.2, 42.8, 31.2, 28.9, 28.2, 27.9, 26.8, 26.8, 22.1, 13.9. HRMS (ESI-TOF): m/z [M+H]⁺ for C₂₈H₄₄N₅⁺ calculé. 450.3591, trouvé 450.3590, err. < 1 ppm.

### Composé PR5 de référence ne faisant pas partie de l'invention

### Composé 21 : N¹-(2,4-dinitro-5-(3,4,5-trimethoxyphenylamino)phenyl)-N⁴-octylbenzene-1,2,4-triamine

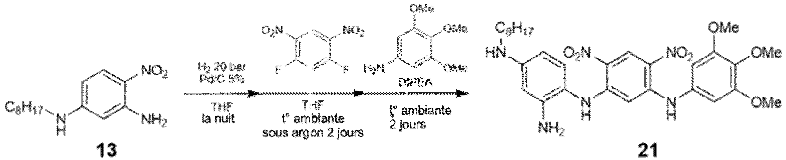

Une solution de compose **13** (1,50 mg, 5,83 mmol, 1,0 eq) dans du THF (50 mL) a été hydrogénée (20 bars) en présence de Pd/C 5% (124 mg, 0,058 mmol, 1 %mol) toute la nuit. Puis le mélange a été dégazé par sonication pendant 5 min. Après ajout de DFDNB (1,10 g, 5,39 mmol, 0,92 eq), La réaction a été agitée à température ambiante sous argon pendant 2 jours. Puis du 3,4,5-trimethoxylaniline (2,20 g, 12, mmol, 2,23 eq) et du DIPEA (600 µL, 3.44 mmol, 0,64 eq) ont été ajoutés à la réaction. Le mélange a été maintenu deux jours supplémentaires à température ambiante. Le Pd/C a été retiré par filtration au travers de Celite. Après concentration sous vide, le produit brut a été repris dans de l'EtOH. Le filtrat a été concentré et purifié par chromatographie sur colonne (silice 60F, AE/CH, 40/60) pour obtenir le composé **21** (1,34 g, 2,30 mmol, 43% de rendement) sous la forme d'un solide rouge. ¹H NMR (400 MHz, CDCl₃): δ 9.73 (br s, 1H), 9.31 (s, 1H), 9.15 (br s, 1H), 6.75 (d, *J =* 8.4 Hz, 1H), 6.37 (s, 2H), 6.28 (s, 1H), 5.95 (dd, *J =* 8.4, 2.5 Hz, 1H), 5.90 (d, *J =* 2.5 Hz, 1H), 3.79 (s, 3H), 3.76 (s, 6H), 3.61 (br s, 2H), 3.58 (br s, 1H), 3.00 (t, *J =* 7.2 Hz, 2H), 1.58 (quintet, *J* = 7.2 Hz, 2H), 1.40-1.29 (m, 10H), 0.90 (t, *J* = 6.9 Hz, 3H). ¹³C NMR (100 MHz, CDCl₃): δ 153.7, 149.4, 149.0, 146.7, 143.7, 136.3, 133.0, 129.3, 128.7, 125.23, 125.16, 111.8, 104.3, 101.7, 98.6, 95.4, 60.9, 56.1, 43.9, 31.8, 29.49, 29.39, 29.25, 27.2, 22.6, 14.1. HRMS (ESI-TOF): m/z [M+H]⁺ for C₂₉H₃₉N₆O₇⁺ calculé. 583.2875, trouvé 583.2878, err. < 1 ppm.

### Composé PR5 de référence :N⁷-octyl-N²-(3,4,5-trimethoxyphenyl)phenazine-2,3,7-triamine

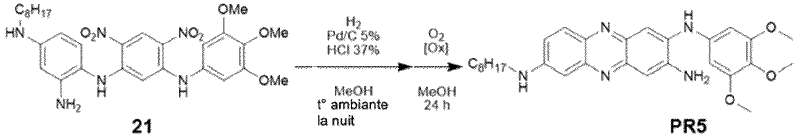

Une solution de composé **21** (150 mg, 0,257 mmol) dans du MeOH (75 mL) a été hydrogénée (20 bars) en présence de Pd/C (5%) et d'HCl (12M, 0,5 mL) toute la nuit. Après ajout de MeOH (30 mL), La solution a été agitée sous air pendant 24 h. Le Pd/C a été retiré par filtration sous Celite, et la phase solide a été rincée avec du MeOH (400 mL). La solution a été neutralisée avec du NaHCO₃ jusqu'à pH 9 avant d'ajouter de l'eau (100 mL). Le solvant a été concentré jusqu'à environ 100 mL sous pression réduite. Après refroidissement à 5 °C toute la nuit, le solide résultant en suspension a été isolé par filtration et lavé avec de l'eau (2 x 30 mL), séché sous vide pour obtenir le composé **PR5** (110 mg, 0.218 mmol, 85% de rendement) sous la forme d'une poudre rouge. ¹H NMR (400 MHz, **DMSO-d₆):** δ 7.79 (d, *J* = 9.2 Hz, 1H), 7.60 (br s, 1H), 7.23 (s, 1H), 7.05 (dd, *J* = 9.2, 2.5 Hz, 1H), 6.89 (d, *J* = 2.5 Hz, 1H), 6.38 (s, 2H), 5.56 (s, 1H), 4.28 (br s, 2H), 4.19 (br t, *J =* 5.3 Hz, 1H), 3.85 (s, 3H), 3.82 (s, 6H), 3.28 (td, *J =* 6.9, 5.5 Hz, 2H), 1.72 (quintet, 7.2 Hz, 2H), 1.48-1.25 (m, 10H), 0.89 (t, *J* = 6.8 Hz, 3H). ¹³C NMR (100 MHz, **DMSO-d₆):** δ 153.3, 148.4, 144.30, 144.17, 142.1, 138.4, 137.0, 136.26, 136.06, 132.5, 128.7, 122.0, 108.2, 103.5, 98.7, 97.8, 60.1, 55.76, 55.59, 42.7, 31.2, 28.82, 28.68, 28.2, 26.7, 22.0, 13.9. HRMS (ESI-TOF): m/z [M+H]⁺ for C₂₉H₃₈N₅O₃⁺ calculé. 504.2969, trouvé 504.2972, err. < 1 ppm.

### 3- Procédé de synthèse n°3 - composé de formule II (1b)

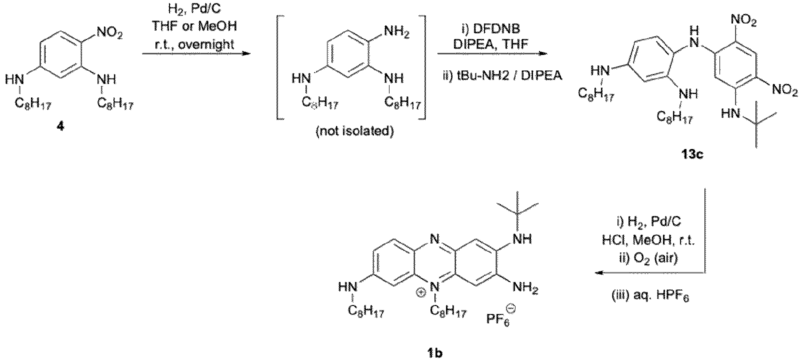

**Composé 13c** : Une solution de composé 4 (625 mg, 1,66 mmol, 1 équiv.) dans du THF (35 mL) a été hydrogénée (40 bars) toute la nuit en présence de Pd/C (5 poids.%, 36 mg, 1 mol%). Après diminution de la pression, la solution a été dégazée par sonication pendant 5 min, puis refroidie à 0°C dans un bac à glace, et du 1,5-difluoro-2,4-dinitrobenzene (320 mg, 1,57 mmol, 0.95 équiv.) a été ajouté à la solution sous agitation. Puis du tert-butylamine (736 µL, 6,98 mmol, 4,2 équiv.) et du DIPEA (602 µL, 3,46 mmol, 2,1 équiv.) ont été ajoutés. Le mélange a été agité à température ambiante pendant 4 jours. Après filtration au travers de celite *via du* dichlorométhane et évaporation de la solution, le produit brut a été purifié par flash chromatographie sur silicagel en utilisant du dichloromethane/cyclohexane (1/1 à 6/4) en tant qu'éluant pour obtenir le composé 13c sous la forme d'un solide rouge (575 mg, 63% de rendement).

**TLC:** R_{f} = 0.25 (SiO₂ F60, dichloromethane/cyclohexane, 7/3). **¹H NMR (400 MHz, CDCl₃):** δ 9.26 (s, 1H), 9.00 (br s, 1H), 8.40 (br s, 1H), 6.86 (d, *J* = 8.3 Hz, 1H), 5.99 (dd, *J* = 8.3, 2.4 Hz, 1H), 5.95 (d, *J* = 2.4 Hz, 1H), 5.90 (s, 1H), 3.81 (br s, 1H), 3.66 (br s, 1H), 3.12 (t, *J* = 7.1 Hz, 2H), 3.07 - 3.03 (m, 2H), 1.66 - 1.59 (m, 2H), 1.56 - 1.50 (m, 2H), 1.44 - 1.22 (m, 29H), 0.89 (t, 6.9 Hz, 3H) , 0.86 (t, 6.9 Hz, 3H). **¹³C NMR (100 MHz, CDCl₃):** δ 149.7, 148.4, 147.4, 145.4, 129.5, 129.0, 125.5, 124.0, 112.1, 101.5, 95.6, 95.1, 52.0, 44.1, 43.5, 31.8, 31.8, 29.5, 29.5, 29.4, 29.3, 29.3, 29.2, 29.0, 27.2, 27.1, 22.7, 22.6, 14.1, 14.1. **HRMS (ESI-TOF):** m/z [M+H]⁺ for C₃₂H₅₃N₆O₄⁺ calculé. 585.4123, trouvé 585.4123, err. < 1 ppm.

**Composé 1b** : Une solution de compose 13c (1 g, 1,71 mmol, 1 équiv.) dans du méthanol (60 mL) a été hydrogénée (20 bars) en présence de Pd/C (5% en masse) te du HCl (12M, 0,5 mL) pendant 6h. Puis le mélange a été agité sous air pendant 16h. Le Pd/C a été retiré par filtration au travers de celite (AW) qui avait été rincée plusieurs fois avec du méthanol et du dichlorométhane. Après retrait du solvant sous pression réduite, le solide résultant a été repris dans dichlorométhane, lavé avec une solution aqueuse de HPF₆ (5% en masse dans de l'eau, 2 x 60 mL) et de l'eau distillée (60 mL). La couche organique a été séchée avec du Na₂SO₄, filtrée et évaporée sous pression réduite. Le résidu finalement obtenu a été précipité dans du pentane et filtré pour obtenir le produit **1b[PF₆⁻]** sous la forme d'un solide rouge (1,05 g, 95% de rendement).

**¹H NMR (250 MHz, CD₃CN, dilué):** δ 7.85 (d, *J* = 9.3 Hz, 1H), 7.24 - 7.20 (m, 2H), 6.99 (s, 1H), 6.57 (d, *J* = 1.8 Hz, 1H), 6.28 - 6.20 (br m, 3H), 4.58 (d, *J* = 8 Hz, 2H), 4.37 (br s, 1H), 3.37 (td, *J =* 7.0 Hz, *J =* 6.2 Hz, 2H), 1.75 - 1.55 (m, 4H), 1.52 (s, 9H), 1.46 - 1.31 (m, 20H), 0.92 - 0.87 (m, 6H). **¹H NMR (250 MHz,** CD₃CN, **concentré):** δ 7.76 (d, *J =* 9.3 Hz, 1H), 7.20 - 7.15 (m, 2H), 6.96 (s, 1H), 6.49 (d, *J =* 1.8 Hz, 1H), 6.34 - 6.29 (br m, 3H), 4.50 (d, *J* = 8 Hz, 2H), 3.33 (m, 2H), 1.76 - 1.30 (m, 33H), 0.91 - 0.86 (m, 6H). **¹³C NMR (63 MHz, CD₃CN, concentré)**:154.3, 152.4, 139.3, 137.8, 137.6, 134.1, 133.2, 131.3,121.7, 109.6, 94.7, 90.3, 53.1, 48.8, 44.2, 32.5, 32.5, 30.0, 30.0, 29.9, 29.2, 29.1, 27.8, 27.5, 27.2, 23.3, 23.3, 14.3, 14.3. HRMS (ESI-TOF): m/z [M+H]⁺ for C₃₂H₅₂N₅⁺ calculé. 506.4217, trouvé 506.4220, err. < 1 ppm.

### Exemple 2 - Propriétés physicochimiques des composés selon l'invention

Les inventeurs ont identifié les différentes propriétés suivantes des composés selon l'invention :

### a. solubilité

La versatilité de la méthode de synthèse permet l'introduction de différents substituants à l'infini permettant de moduler les propriétés de solubilité. Selon la nature hydrophile / hydrophobe de R1, R2, R3 et R4, il est ainsi envisageable de solubiliser les composés de type II dans des solvants polaires, apolaires, protiques ou aprotiques.

On constate que bien que les composés soient plus solubles dans les solvants organiques, ceux-ci présentent de propriété de solubilité dans l'eau, propriété d'un intérêt majeur pour une utilisation in vitro et in vivo sur des échantillons biologiques et cellulaires.

### a. Caractérisation des formes protonées

Les inventeurs ont caractérisé les différentes formes protonées de composés de formule I ou R₁ et R₂ sont C₈H₁₇, R₃ est du tert-butyle et R₄ H, de formule suivante : 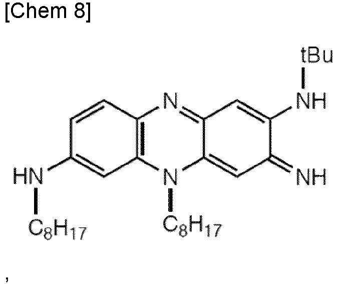 ainsi que les formes mono, di et tri protonées de formules respectives suivantes 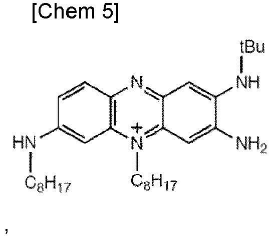 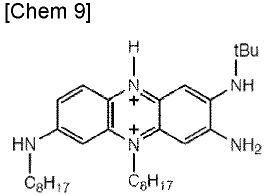 et

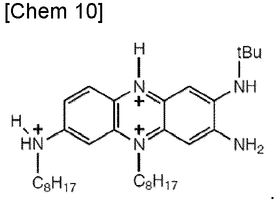

**Les résultats sont présentés à la** **Figure 1****.**

Les spectres RMN ¹H du composé 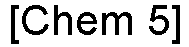 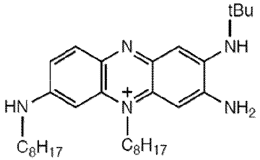 ont confirmé cette hypothèse avec notamment la présence de trois protons, Ha, Hb et Hc, dans la région aromatique avec des constantes de couplage classiquement rencontrées au sein d'un benzène 1,2,4-trisubstitué. Les données RMN ¹H ont également montré la présence de deux chaînes octyles magnétiquement non équivalentes. L'ensemble de ces données suggère la formation d'un dérivé de type phénazinium

En outre, les signaux des protons H_{d} et Hₑ - respectivement à δ = 7,17 et 7,00 ppm - subissent un effet de blindage très important (δ_{Hd} = 6,25 ppm et (δ_{He} = 5,91 ppm) après ajout de NaOD (40% w/w dans D₂O), effet inhabituellement observé au sein d'un système aromatique mais déjà vu au sein de certaines triaminophénazines (δ = 6,5-6,1 ppm et 5,9-5,4 ppm) décrites par Roy.^{[26]} Cette observation peut s'expliquer par une réaction de déprotonation induisant une rupture de l'aromaticité en faveur d'une structure de type quinoïdale

### a. Propriétés optiques

Outre la caractérisation des formes protonnées les inventeurs ont également testé les propriétés d'absorption et d'émission des différents composés selon l'invention.

### i) Propriétés d'absorption

Les inventeurs ont testé l'absorption des quatre composés plus ou moins protonés susmentionnés et évalué ε (M⁻¹cm⁻¹) en fonction de la longueur d'onde λ (en nm).

La Figure. 2 montre le spectre d'absorption de la molécule de type I. Le spectre d'absorption traduit la présence d'une espèce non chargée de type I pour laquelle le degré de délocalisation / conjugaison est plus faible (effet hypsochrome) que pour les espèces cationiques de type II.

La Figure 3 montre le spectre d'absorption de la molécule de type II. Le spectre d'absorption traduit la présence d'une espèce mono chargée de type II pour laquelle le degré de délocalisation / conjugaison est plus important (effet bathochrome) que pour l'espèce neutre de type I.

La Figure 4 montre le spectre d'absorption de la molécule de type C. Un *« super »* acide, l'acide triflique (HOTf) (pKa_{(MeCN)} = 0,70^{[31]}), a été utilisé pour protoner Il dans la cellule UV (C ≈ 1,44.10⁻⁵ M, dans MeCN). Lors de l'ajout d'acide (de 0,1 à 16 équiv.), l'intensité des bandes initiales situées à 267, 308, 465 et 552 nm diminuent au profit de l'apparition de nouvelles bandes à 274, 322, 507 et 686 nm. Cette évolution spectrale traduit la disparition du composé de départ **II** au profit de la formation d'une seule espèce de type C dont les bandes d'absorption sont de plus haute énergie. La première étape de protonation est terminée après l'ajout de 16 équiv. d'HOTf

La Figure 5 montre le spectre d'absorption de la molécule de type D. Au-delà de l'ajout de 16 équiv. d'acide triflique, une nouvelle espèce apparaît (nouvelles bandes à 268, 287, 370, 475 et 506 nm) en même temps que disparaît le cation C. La double protonation de Il est terminée après l'ajout de plus 2600 équiv. d'HOTf. La bande de C à 686 nm a complètement disparu au profit du spectre d'un trication D possédant une bande étroite à 506 nm et muni d'un épaulement caractéristique d'une structure de type cyanine.

### ii) Propriétés d'émission

Les inventeurs ont testé l'émission de fluorescence en fonction de la longueur d'onde λ (en nm) pour le composé suivant repris dans de l'acétonitrile

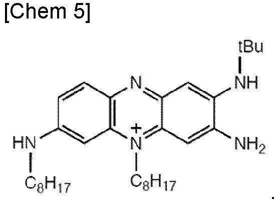

### Les résultats sont présentés à la Figure 6.

Le spectre d'absorption UV-Vis de **II** a montré la présence de deux bandes principales situées à λₘₐₓ = 265nm et 549 nm et avec des épaulements respectifs situés à λ = 295 nm et 578 nm. La molécule Il est également fluorescente et émet à λₑₘ = 642 nm (excitation à À = 550 nm). Cette émission est comparable à celle produite par le composé analogue cationique « *rouge neutre* » reporté dans la littérature (λ_{abs} = 534 nm et λₑₘ = 616 nm)

### iii) rendement de fluorescence et émission

Les inventeurs se sont ensuite attachés à mesurer la valeur Φf le rendement quantique de fluorescence le composé suivant repris dans de l'acétonitrile

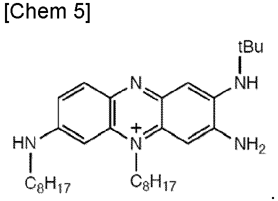

Pour ce faire, les inventeurs ont mesuré l'absorbance (intensité relative) en fonction de la longueur d'onde (en nm) en présence de doses croissantes de 1,8-DiazaBicyclo[4.3.0]Undéc-7-ène (DBU - 0 à 2 équivalents).

### Les résultats sont présentés en Figure 7.

Le phénazinium **II** et sa base conjuguée **II-H** sont fluorescents dans le MeCN à pH neutre ou basique. En revanche, aucune propriété de luminescence n'a été observée en milieu acide. La Fig. 7 présente l'évolution spectrale du phénazinium **II** lors de l'ajout de DBU (excitation à 483 nm). Cette évolution traduit clairement la disparition du composé de départ (diminution de la bande à 637 nm ) au profit de la formation d'une seule espèce **II-H** possédant une émission à plus haute énergie beaucoup plus faible que celle de **II** (augmentation de la bande à 550 nm). Les calculs de rendements quantiques montrent en effet que la forme déprotonée **[II-H]** produit moins de fluorescence (Φ_{f} = 0,08 à 550 nm) que la forme de départ II (Φ_{f} = 0,76 à 637 nm). La référence utilisée pour le calcul du rendement quantique de fluorescence est la tétraphénylporphyrine dans l'acétonitrile (Φ_{f} = 0,15).

Le maximum d'émission se situe à une longueur d'onde localisée dans le rouge lointain à 645 nm.

Le coefficient Φf trouvé est de 0,76, ce qui témoigne d'un rendement de fluorescence très élevé, et une forte brillance d'environ 50000.

La brillance (B) est proportionnelle à la quantité de lumière émise par fluorescence à une lumière d'excitation donnée selon la relation B = ε x Φ (avec ε = coefficient d'extinction molaire et Φ le rendement quantique d'émission).

Le calcul de ε est effectué à l'aide spectrophotomètre mesurant l'absorbance A (grandeur sans unité) d'une solution diluée de concentration C connue dans une cuve d'épaisseur I.

La relation fondamentale utilisée en spectrophotométrie est présentée sous la forme : A = ε . I . c (A étant l'absorbance ou densité optique)

Calcul de Φ : Il est déterminé en mesurant l'intensité de l'émission d'une solution de concentration connue, la référence utilisée pour le calcul du rendement quantique de fluorescence est ici la tétraphénylporphyrine dans l'acétonitrile (ϕ_{f} = 0,15).

Le rendement quantique est défini par :
Φ = nombre de photons émis / nombre de photons absorbés

### iv) fluorescence in vivo

Au préalable des tests de marquage fluorescents sur des lignées cellulaires, les inventeurs ont testé la toxicité des composés selon l'invention.

Des lignées cancéreuses MCF-7 ont été ensemencées dans des plaques 96-puits à une concentration d'environ 5000 cellules/puits dans 200 µL de milieu de culture et laissées en culture pendant 24h. Ensuite, les cellules ont été incubées pendant 72 h, avec ou sans le compose à tester (de 1 nM à 1 µM). Après incubation avec les composés, un test MTT a été réalisé afin de tester la cytotoxicité des composés. Brièvement, les cellules ont été incubées en présence de 0.5 mg mL⁻¹ de MTT pendant 4 h afin de mesurer l'activité mitochondriale. Puis, les précipités de MTT ont été dissouts dans 150 µL d'une solution de mélange d'ethanol/DMSO (1:1) et l'absorbance a été lue à 540 nm.

Les inventeurs sont arrivés à la conclusion que la dose 100 nM n'affectait pas significativement la survie des cellules, comme en témoigne la **Figure 14****.** On observe en effet très nettement que la cytotoxicité dans le noir est faible à une concentration de 100 nM (Fig. 14)

Les inventeurs ont testé la fluorescence du composé de formule suivante 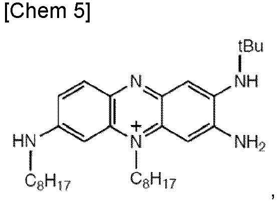 sur des cellules de cancer du sein MCF-7 en microscopie monophotonique (excitation à 514 nm) ou biphotonique (excitation à 790 ou 810 nm).

### Imagerie à un ou deux photons

Les cellules de cancer du sein humain MCF-7 ont été ensemencées dans des boites de pétri (World Precision Instrument, Stevenage, UK) disposant d'une plaque de verre dans le fond, dans 2 mL de milieu de culture. Les cellules ont ensuite été incubées pendant 16 h avec le compose selon l'invention à une concentration de 0.1 µM ou de 0.5 µM. 15 minutes avant la fin de l'incubation, les cellules ont été incubées avec du Hoechst 33342 (Invitrogen, Cergy Pontoise, France) à une concentration finale de 5 µg.mL⁻¹ afin de marquer les noyaux des cellules. Ensuite, les cellules ont été lavées deux fois avec du milieu de culture.

L'imagerie par fluorescence à un photon a été réalisée sur des cellules vivantes à une longueur d'onde 514 nm à l'aide d'un microscope Confocal Carl Zeiss (LSM780). L'imagerie par fluorescence à deux photons a été réalisée à des longueurs d'onde de 790 nm ou de 810 à l'aide du laser Chameleon disponible sur le même microscope. Toutes les images ont été réalisées avec le même objectif, au même grossissement (63×/1.4 OIL DIC Plan-Apo).

Les résultats obtenus en microscopie monophotonique sont présentés à la **Figure 8****,** et en microscopie biphotonique à la **Figure 9****.**

Ces composés ont montré des propriétés remarquables d'imagerie à un et deux photons (Fig. 3 et 4). Il apparaît clairement que les composés sont facilement identifiables et que leur localisation n'est que cytoplasmique (les co-localisations sont parfaitement concluantes). Il est intéressant de noter que les marquages les plus nets et les plus intenses sont obtenus avec les plus faibles concentrations. En effet, les marquages sont nettement plus fins et laissent apparaître des granules cytoplasmiques intenses, ce qui tend à montrer qu'une identification plus précise des cibles cytoplasmiques est possible dans ces conditions. Notamment, d'intenses foyers de fluorescence obtenus dans des régions périnucléaires peuvent correspondre au réticulum endoplasmique.

Les inventeurs ont également testé l'internalisation des composés selon l'invention au cours du temps.

La cinétique d'internalisation a été réalisée un lecteur CLARIOstar afin de quantifier l'internalisation du composé dans les cellules tumorales MCF-7. Les valeurs, correspondant au rapport de la fluorescence résiduelle / la fluorescence totale sont présentés sous la forme de moyennes de trois expériences, ± l'écart type.

Il apparaît clairement (Fig. 10) que 10% du composé fluorescent (II) pénètre la cellule en moins de 24h, permettant d'observer des images de très grandes qualités.

La cinétique d'incorporation par les cellules MCF-7 est montrée en **Figure 10****.**

### a. Production ¹O₂

Les spectres d'absorption ont été mesurés avec un spectrophotomètre double faisceau UV-visible Perkin-Elmer (Lambda EZ 210). Le spectre de fluorescence a été mesuré avec un spectrofluorimètre Fluorolog FL3-222 (Horiba Jobin Yvon, Longjumeau, France) équipé d'une lampe à arc xénon de 450 W, d'un compartiment thermostaté (25°C), d'un photomultiplicateur UV-visible R928 (HAMAMATSU Japon) et d'un détecteur infrarouge InGaAs refroidi à l'azote liquide (DSS-16A020L Electro-Optical System Inc, Phoenixville, PA, USA). Le faisceau d'excitation est séparé par un monochromateur double réseaux SPEX (1200 traits/mm blasé à 330 nm). La fluorescence a été mesurée par le détecteur UV-Visible via le monochromateur d'émission double réseaux SPEX (1200 traits/mm blasé à 500 nm). La production d'oxygène singulet a été mesurée par le détecteur infrarouge via le monochromateur d'émission double réseaux SPEX (600 traits/mm blasé à 1 µm). Tous les spectres ont été mesurés en utilisant des cuves quartz à 4 faces. Les valeurs d'absorption à la longueur d'onde d'excitation des références et des échantillons ont été ajustées à environ 0,2.

Par cette méthode, les inventeurs ont pu mesurer le rendement quantique Φ_{Δ} qui est de 0,11 pour le composé

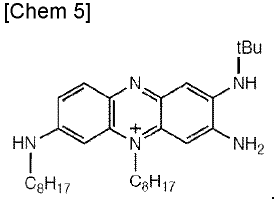

Ce faible rendement est attendu, puisque la grande majorité des photons absorbés est convertie en lumière, environ 76% des photons.

### Exemple 3 - Utilisation des composés selon l'invention en thérapie photodynamique in vitro

Les inventeurs ont testé l'effet des composés selon l'invention en thérapie photodynamique. Des cellules MCF-7 ont été incubées avec le composé de formule 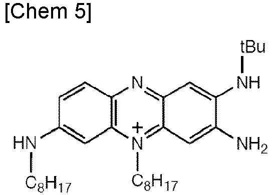 pendant 5 h et irradiées (ou non) à 530 nm pendant 20 minutes. Deux jours plus tard, les cellules furent soumises à un test colorimétrique de viabilité cellulaire (MTT).

Les résultats sont présentés aux **Figures 12** et **13****.**

Les résultats obtenus sous irradiation à un photon (λ_{irrad}. = 514 nm) sont exceptionnels puisque 98% des cellules tumorales sont tuées à des concentrations très faibles (C=100 nM).

Comme on le constate sur la figure 13, à la concentration de 100 nM, deux zones sont facilement distinguées grâce aux cristaux violets du MTT qui ne colorent que les cellules vivantes (non irradiées).

La thérapie photodynamique biphotonique (λ_{irrad}. = 810 nm) a montré des résultats très encourageants puisque près de 50% des cellules tumorales sont tuées, sans optimisation (irradiation pendant seulement 5 secondes à une concentration très faible de 100 nM de composé selon l'invention).

Les inventeurs ont également testé l'effet des composés selon l'invention en thérapie photodynamique sur d'autres modèles. Des tests de traitements de kératose ont été réalisés à différentes concentrations d'un composé photosensibilisateur (PS) : soit celui utilisé sur les cellules MCF-7, soit le composé de formule 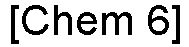 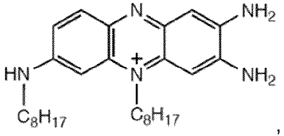 sur des kératinocytes en culture. Le PS a été ajouté aux cellules pendant 20 minutes à des doses de 0 nM (A), 10 nM (B), 25 nM (C) ou 50 nM (D) et ces dernières ont été irradiées (colonnes noires) ou non (colonnes grises) à 530 nm **(****Figures 15** **et** **17****).** Deux jours plus tard, les cellules sont soumises à un test colorimétrique de viabilité cellulaire (MTT) **(****Figures 16 et 18****).**

### Exemple 4 - Internalisation des composés selon l'invention

Les inventeurs ont évalué la capacité des cellules à internaliser les composés selon l'invention.

Pour ce faire des cellules MCF-7 ou des fibroblastes de donneur sains ont été traités ou non avec 0,5 nM de composé de formule 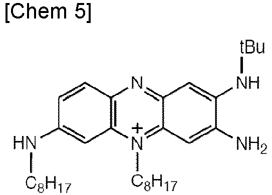 pendant 1, 3, 6 ou 24 h, et la fluorescence des cellules celles a été évaluée en cytométrie de flux en détectant le nombre de cellules ayant une fluorescence rouge.

Les résultats sont visibles aux **Figures 20** **et** **21****.**

A concentration égale (0,5 nM) après 6 h d'incubation avec le composé selon l'invention, 46% des cellules cancéreuses (MCF-7) ont internalisé le composé mais seulement 18% des cellules saines (fibroblastes). De la même façon, 90% des cellules cancéreuse ont internalisé le composé selon l'invention après 24 h contre seulement 24% pour les cellules saines.

Ces résultats montrent que le composé selon l'invention entre plus rapidement dans les cellules cancéreuses que dans les cellules saines.

### Exemple 5 - Comparaison absorption phénazinium selon l'invention et phénazine décrits dans l'art antérieur

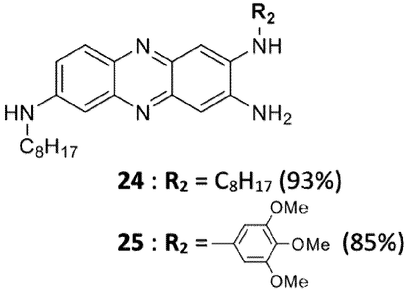

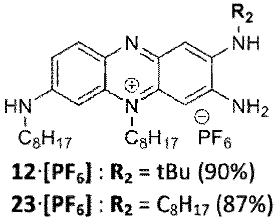

Le phénaziniums cationiques ([12]⁺ ; [23]⁺) sont beaucoup plus solubles que les phénazines neutres (24 et 25). Ces dernières sont en effet insolubles dans les alcools et peu solubles (C < 10⁻⁴ M) dans le MeCN, ou l'acétone alors que les phénaziniums cationiques ([12]⁺ ; [23]⁺) sont bien solubles dans tous les solvant communs (i.e. toluène, Et₂O, CH₂CL₂, CHCl₃, acétone, MeCN, MeOH, EtOH DMF, DMSO) en raison de leur caractère amphiphile (la partie chargée étant hydrophile et la partie alkylée étant hydrophobe).

Les spectres d'absorption des composés ([12]⁺ ; [23]⁺) sont presque identiques et présentent une bande située dans la région du visible à λₘₐₓ = 553 nm (ε⁵⁴³ = 43400 M⁻¹cm⁻¹ et ε⁵⁴³ = 41200 M⁻¹cm⁻¹) avec un épaulement vers 465 nm. Deux bandes d'absorption situées dans l'ultra-violet à 265 nm et 320 nm viennent compléter ces spectres d'absorption.

Les caractéristiques d'absorption des composés phénazines 24 et 25 sont similaires à celles des composés phénaziniums cationiques avec toutefois un décalage de 100 nm vers le bleu, leur absorption apparaissant respectivement à λₘₐₓ = 472 nm (ε⁴⁷² = 16100 M⁻¹cm⁻¹) et λₘₐₓ = 472 nm (ε⁴⁷² = 14800 M⁻¹cm⁻¹). Les intensités correspondantes sont en revanche beaucoup plus faibles (35 à 45% de l'intensité du pic principal des composés phénaziniums cationiques). Par ailleurs, les composés 24 et 25 montrent plusieurs bandes d'absorption supplémentaires situées entre 220 et 300 nm.

### Les données sont présentées en Figure 19.

Ceci démontre que les composés phénaziniums sont plus appropriés pour de la thérapie photonique que les phénazines neutres, car ils sont plus solubles et ils peuvent être irradiés avec un laser à plus faible énergie (longueurs d'ondes d'excitation plus grande).

## Revendications

1. Composé de formule I suivant : 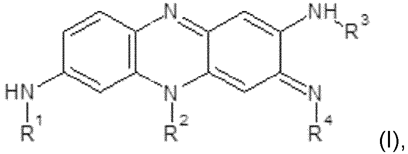 où,
indépendamment l'un de l'autre, R1 et R2 sont un alkyle en C4-C10, linéaire ou ramifié, saturé ou non, cyclique ou non, et
indépendamment l'un de l'autre, R3 et R4 sont
- ou H,
- ou un alkyle en C1-C18, linéaire ou ramifié, saturé ou non, cyclique ou non, éventuellement substitué par un ou plusieurs groupe choisi parmi un groupe hydroxyl, un groupe amino, un groupe aminoalkyle, un groupe alkoxy en C1-C5, un alkyl en C1-C5, un peptide, un groupe pyridine, un groupe phosphine, un thiol, un C2 alcène, un groupe C2 alcyne et un halogène, or un radical benzyle éventuellement substitué par un ou plusieurs radicaux choisi parmi un groupe hydroxyl, un groupe amino, un groupe aminoalkyl, un groupe C1-C5 alkoxy, un groupe C1- C5 alkyl, and un halogène,
- ou un groupe (hétéro)aryle, éventuellement substitué par un ou plusieurs groupe choisi parmi un groupe hydroxyl, un groupe amino, un groupe aminoalkyle, un groupe alkoxy en C1-C5, un alkyl en C1-C5, un peptide, un groupe pyridine, un groupe phosphine, un thiol, un C2 alcène, un groupe C2 alcyne et un halogène, or un radical benzyl éventuellement substitué par un ou plusieurs radicaux choisi parmi un groupe hydroxyl, un groupe amino, un groupe aminoalkyl, un groupe C1-C5 alkoxy, un groupe C1- C5 alkyl, and un halogène,
- ou encore R3 et R4 sont l'un ou l'autre, ou les deux, un groupe fonctionnel carbonyle formant des fonctions amines incluant des peptides ou non ;
ou un sel ou un solvate de celui-ci, ou une forme protonée de celui-ci.

2. Composé selon la revendication 1, où la forme protonée du composé de formule 1 est choisie parmi les composés suivants :
- le composé de formule la : 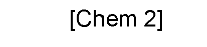 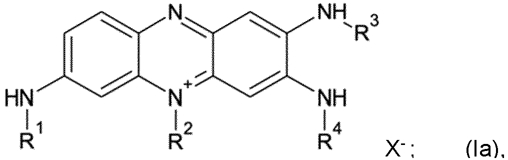
- le composé de formule Ib : 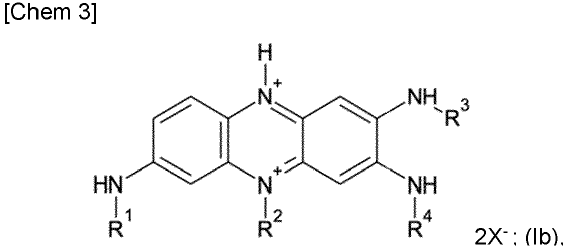 et
- le composé de formule Ic : 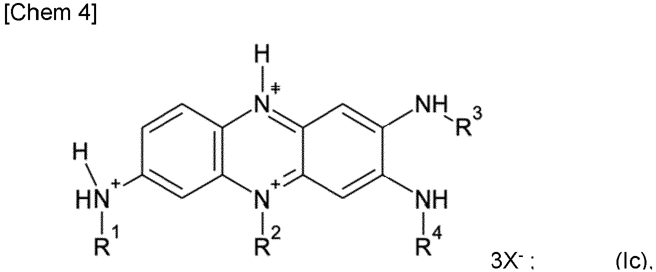 où X représente Cl, Br, OH, F, I, BF₄ ou PF₆ ou trifluorométhanesulfate (Otf).

3. Composé selon la revendication 1 ou 2, le dit composé ayant la formule II, III ou IV suivante : 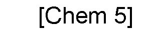 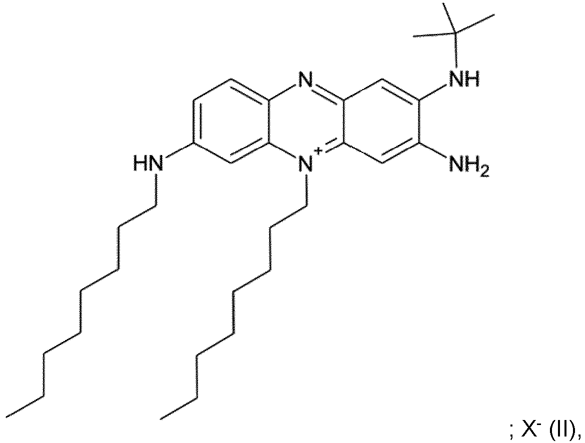 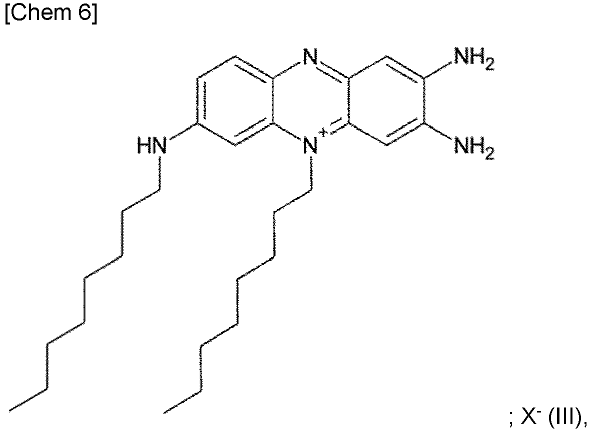 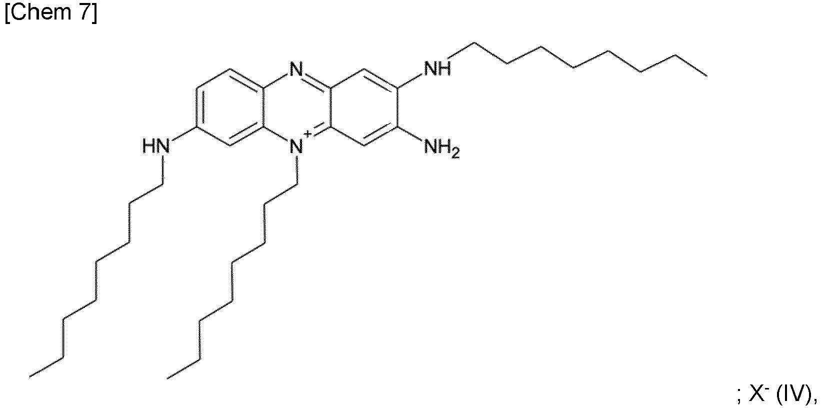 où X représente CI, Br, OH, F ou I.

4. Composition pharmaceutique comprenant, à titre de substance active, un composé selon l'une quelconque des revendications 1 à 3, en association avec un véhicule pharmaceutiquement acceptable.

5. Composé selon l'une quelconque des revendications 1 à 3, pour son utilisation en tant que médicament.

6. Composé selon l'une quelconque des revendications 1 à 3, pour son utilisation pour le traitement de pathologies par thérapie photodynamique.

7. Composé pour son utilisation selon la revendication 6, où lesdites pathologies sont des tumeurs.

8. Composé selon l'une quelconque des revendications 1 à 3, pour son utilisation dans le cadre du diagnostic de pathologies, notamment de cancers.

9. Utilisation d'un composé selon l'une quelconque des revendications 1 à 3, pour la visualisation, in vitro ou ex vivo, de cellules vivantes, d'organites du cytoplasme ou de tissus, par microscopie à fluorescence à un ou deux photons, ledit composé étant notamment utilisé à une concentration variant de 2 à 500 nmol. L-1.

10. Méthode in vitro d'éradication de cellules comprenant une étape d'exposition, à l'aide d'une source lumineuse émettant un ou deux photons, de cellules traitées avec un composé selon l'une quelconque des revendications 1 à 3, ledit composé étant utilisé à une concentration variant de 1 à 1000 nmol. L-1.

## Patentansprüche

1. Verbindung der folgenden Formel I: 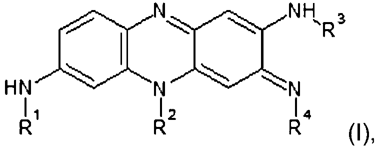 wobei
unabhängig voneinander R1 und R2 ein geradkettiges oder verzweigtes, gesättigtes oder ungesättigtes, cyclisches oder nicht-cyclisches C4-C10-Alkyl sind, und
unabhängig voneinander R3 und R4
- oder H
- entweder ein geradkettiges oder verzweigtes, gesättigtes oder ungesättigtes, cyclisches oder nicht-cyclisches C1-C18-Alkyl, das gegebenenfalls durch eine oder mehrere Gruppen substituiert ist, die ausgewählt sind aus einer Hydroxylgruppe, einer Aminogruppe, einer Aminoalkylgruppe, einer C1-C5-Alkoxygruppe, einem C1-C5-Alkyl, einem Peptid, einer Pyridingruppe, einer Phosphingruppe, einem Thiol, einem C2-Alken, einer C2-Alkingruppe und einem Halogen, oder ein Benzylrest, das gegebenenfalls durch ein oder mehrere Reste, ausgewählt aus einer Hydroxylgruppe, einer Aminogruppe, einer Aminoalkylgruppe, einer C1-C5-Alkoxygruppe, einer C1-C5-Alkylgruppe und einem Halogen, substituiert ist,
- oder (Hetero)arylgruppen, die gegebenenfalls durch eine oder mehrere Gruppen aus der Gruppe Hydroxylgruppe, Aminogruppe, Aminoalkylgruppe, C1-C5-Alkoxygruppe, C1-C5-Alkylgruppe, Peptidgruppe, Pyridingruppe, Phosphingruppe, Thiolgruppe, einer C2-Alkenylgruppe oder einer C2-Alkinylgruppe, und einem Halogen, oder einem Benzylrest, der gegebenenfalls durch einen oder mehrere Reste, ausgewählt aus einer Hydroxylgruppe, einer Aminogruppe, einer Aminoalkylgruppe, einer C1-C5-Alkoxygruppe, einer C1-C5-Alkylgruppe und einem Halogen, substituiert ist,
- oder R3 und R4 entweder eine oder beide eine Carbonylfunktionsgruppe bilden, die Aminfunktionen einschließlich Peptide bildet oder nicht;
oder ein Salz oder Solvat davon oder eine protonierte Form davon.

2. Verbindung gemäß Anspruch 1, wobei die protonierte Form der Verbindung der Formel 1 aus den folgenden Verbindungen ausgewählt ist:
- die Verbindung der Formel la: 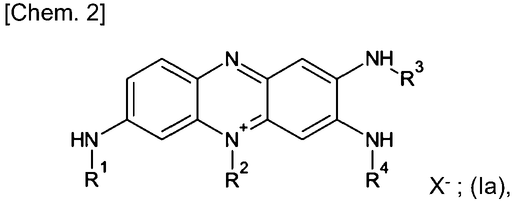
- die Verbindung der Formel Ib: 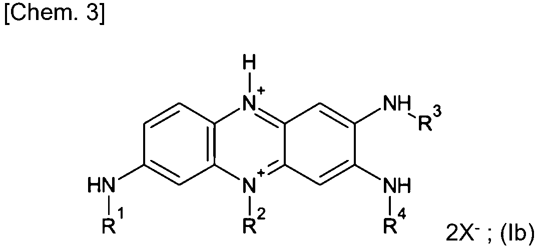 und
- die Verbindung der Formel Ic: 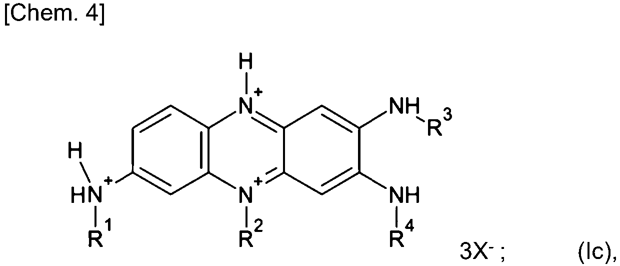 wobei X für Cl, Br, OH, F, I, BF₄ oder PF₆ oder Trifluormethansulfonat (Otf) steht.

3. Verbindung gemäß einem der Ansprüche 1 oder 2, wobei die Verbindung die folgende Formel II, III oder IV aufweist: 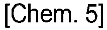 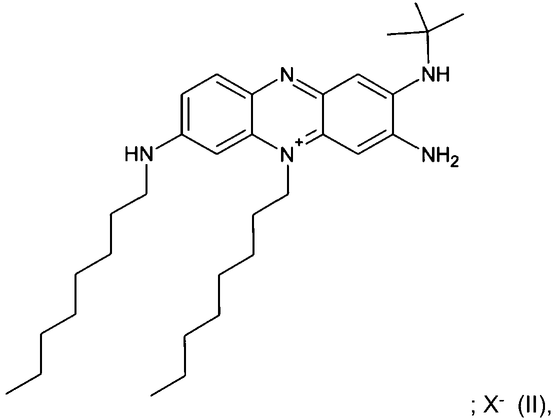 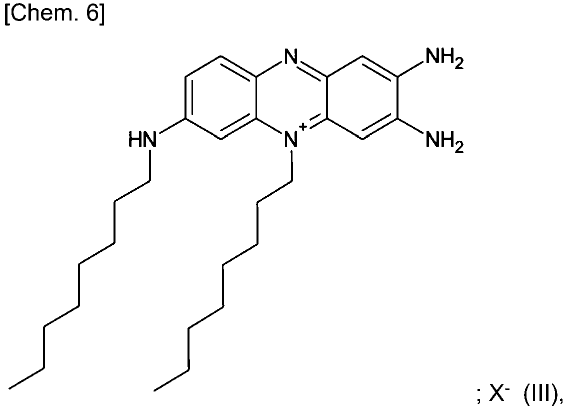 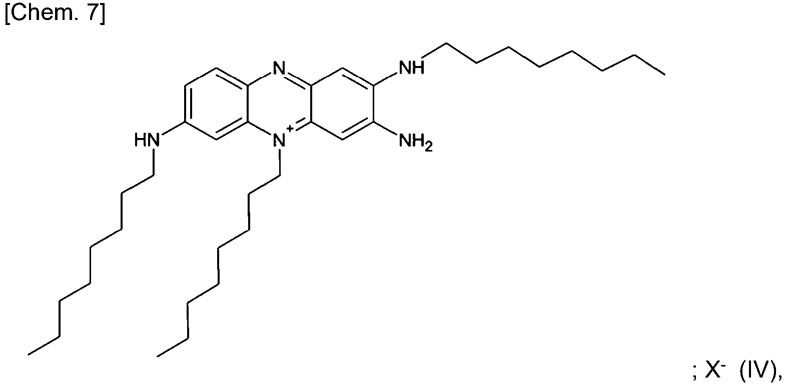 wobei X für Cl, Br, OH, F oder I steht.

4. Pharmazeutische Zusammensetzung, die als Wirkstoff eine Verbindung gemäß einem der Ansprüche 1 bis 3 in Verbindung mit einem pharmazeutisch akzeptablen Hilfsstoff umfasst.

5. Verbindung gemäß einem der Ansprüche 1 bis 3 zur Verwendung als Arzneimittel.

6. Verbindung gemäß einem der Ansprüche 1 bis 3 zur Verwendung bei der Behandlung von Pathologien durch photodynamische Therapie.

7. Verbindung zur Verwendung gemäß Anspruch 6, wobei es sich bei den Pathologien um Tumore handelt.

8. Verbindung gemäß einem der Ansprüche 1 bis 3 zur Verwendung bei der Diagnose von Pathologien, insbesondere Krebserkrankungen.

9. Verwendung einer Verbindung gemäß einem der Ansprüche 1 bis 3 zur *In-vitro-*oder Ex-vivo-Visualisierung lebender Zellen, zytoplasmatischer Organellen oder Gewebe mittels Ein- oder Zwei-Photonen-Fluoreszenzmikroskopie, wobei die Verbindung insbesondere in einer Konzentration von 2 bis 500 nmol.L-1 verwendet wird.

10. In-vitro-Verfahren zur Eliminierung von Zellen, umfassend einen Schritt des Bestrahlens von mit einer Verbindung gemäß einem der Ansprüche 1 bis 3 behandelten Zellen unter Verwendung einer Lichtquelle, die ein oder zwei Photonen emittiert, wobei die Verbindung in einer Konzentration von 1 bis 1000 nmol.L-1 verwendet wird.

## Claims

1. Compound of the following formula I: 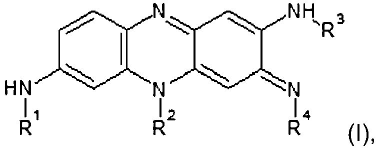 where,
independently of each other, R1 and R2 are a linear or branched, saturated or unsaturated, cyclic or non-cyclic C4-C10 alkyl, and
independently of each other, R3 and R4 are
- or H
- either a linear or branched, saturated or unsaturated, cyclic or non-cyclic C1-C18 alkyl, optionally substituted by one or more groups chosen from a hydroxyl group, an amino group, an aminoalkyl group, a C1-C5 alkoxy group, a C1-C5 alkyl, a peptide, a pyridine group, a phosphine group, a thiol, a C2 alkene, a C2 alkyne group and a halogen, or a benzyl radical optionally substituted by one or more radicals chosen from a hydroxyl group, an amino group, an aminoalkyl group, a C1-C5 alkoxy group, a C1-C5 alkyl group, and a halogen,
- or (hetero)aryl groups, optionally substituted by one or more groups chosen from a hydroxyl group, an amino group, an aminoalkyl group, a C1-C5 alkoxy group, a C1-C5 alkyl, a peptide, a pyridine group, a phosphine group, a thiol, a C2 alkene, a C2 alkyne group and a halogen, or a benzyl radical optionally substituted by one or more radicals chosen from a hydroxyl group, an amino group, an aminoalkyl group, a C1-C5 alkoxy group, a C1-C5 alkyl group, and a halogen,
- or R3 and R4 are either one or both a carbonyl functional group forming amine functions including peptides or not;
or a salt or solvate thereof, or a protonated form thereof.

2. Compound according to claim 1, wherein the protonated form of the compound of formula 1 is chosen from the following compounds:
- the compound of formula la: 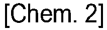 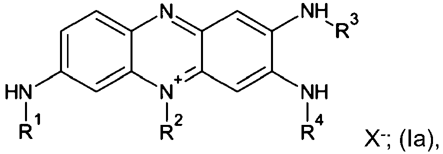
- the compound of formula Ib: 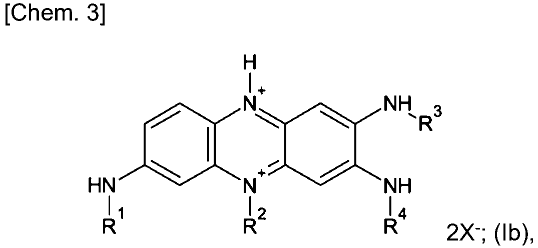 and
- the compound of formula Ic: 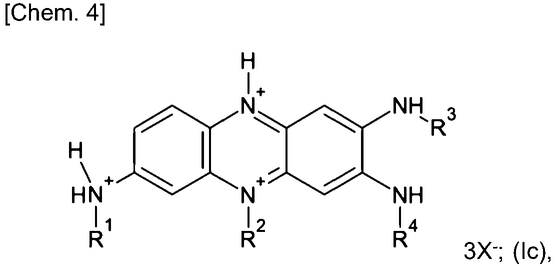 where X represents Cl, Br, OH, F, I, BF₄ or PF₆ or trifluoromethanesulfate (Otf).

3. Compound according to one of claims 1 or 2, said compound having the following formula II, III or IV: 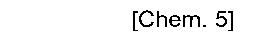 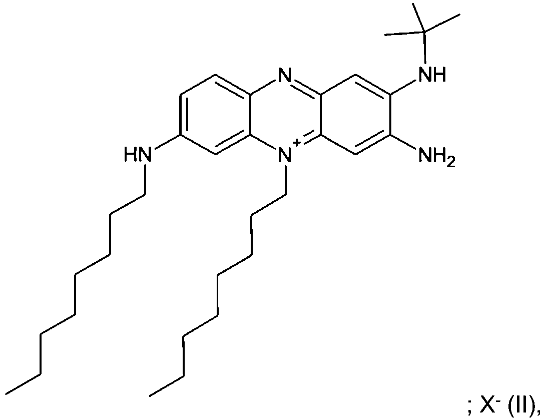 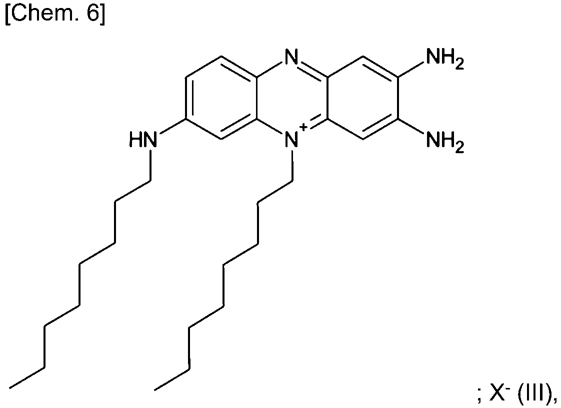 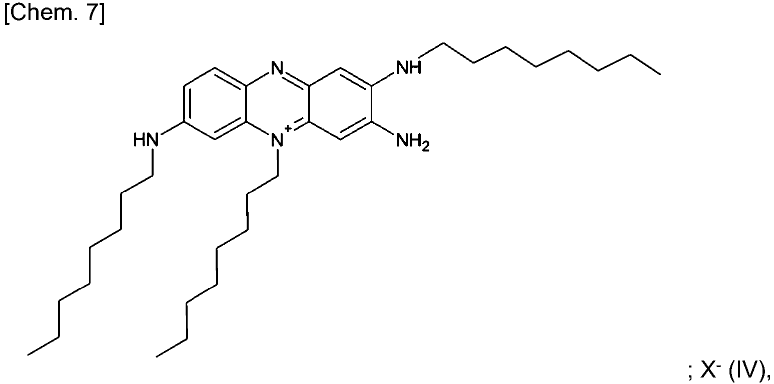 where X represents Cl, Br, OH, F or I.

4. Pharmaceutical composition comprising, as active substance, a compound according to any one of claims 1 to 3, in association with a pharmaceutically acceptable vehicle.

5. Compound according to any one of claims 1 to 3, for use thereof as a drug.

6. Compound according to one of claims 1 to 3, for use in the treatment of pathologies by photodynamic therapy.

7. Compound for use according to claim 6, wherein said pathologies are tumors.

8. Compound according to any one of claims 1 to 3, for use in the diagnosis of pathologies, particularly cancers.

9. Use of a compound according to one of claims 1 to 3, for the *in vitro* or *ex vivo* visualization of living cells, of cytoplasmic organelles or of tissues, by one- or two-photon fluorescence microscopy, said compound being used in particular at a concentration varying from 2 to 500 nmol.L-1.

10. *In vitro* method for eradicating cells comprising a step of exposing, using a light source emitting one or two photons, cells treated with a compound according to any one of claims 1 to 3, said compound being used at a concentration varying from 1 to 1000 nmol.L-1.
